# EUROPEAN PATENT SPECIFICATION

(11) **EP 2 349 287 B1**
(45) Date of publication and mention of the grant of the patent: **15.08.2018**
(21) Application number: 09788170.0
(22) Date of filing: 11.03.2009
(51) Int. Cl.: A61K 31/7105, C12N 5/10, A61K 48/00, A61P 21/00

(54) **METHODS AND MEANS FOR EFFICIENT SKIPPING OF AT LEAST ONE OF THE FOLLOWING EXONS OF THE HUMAN DUCHENNE MUSCULAR DYSTROPHY GENE: 43, 46, 50- 53.**
VERFAHREN UND MITTEL ZUM EFFIZIENTEN ÜBERSPRINGEN ZUMINDEST EINES DER FOLGENDEN EXONE DES MENSCHLICHEN GENS FÜR DUCHENNE-MUSKELDYSTROPHIE: 43, 46, 50-53
MÉTHODES ET MOYENS POUR SAUTER EFFICACEMENT AU MOINS L`UN DES EXONS SUIVANTS DU GÈNE DE LA DYSTROPHIE MUSCULAIRE HUMAINE DE DUCHENNE : 43, 46, 50 À 53

(30) Priority: 27.10.2008 WO PCT/NL2008/050673
(43) Date of publication of application: 03.08.2011
(60) Divisional application: 18180952.6
(73) Proprietor: Academisch Ziekenhuis Leiden, 2333 ZA Leiden (NL); BioMarin Technologies B.V., 2333 CH Leiden (NL)
(72) Inventor: DE KIMPE, Josephus Johannes, 3521 BE Utrecht (NL); PLATENBURG, Gerardus Johannes, NL-2252 XR Voorschoten (NL); VAN DEUTEKOM, Judith Christina Theodora, NL-3329 AA Dordrecht (NL); AARTSMA-RUS, Annemieke, NL-2134 BE Hoofddorp (NL); VAN OMMEN, Garrit-Jan Boudewijn, NL-1015 LW Amsterdam (NL)
(74) Representative: Nederlandsch Octrooibureau
(86) International application number: PCT/NL2009/050113
(87) International publication number: WO 2010/050802

(56) References cited:
- EP-A1- 1 857 548
- WO-A1-2006/000057
- WO-A2-2009/054725
- AARTSMA-RUS ANNEMIEKE ET AL: "Functional analysis of 114 exon-internal AONs for targeted DMD exon skipping: indication for steric hindrance of SR protein binding sites" OLIGONUCLEOTIDES, MARY ANN LIEBERT, NEW YORK, NY, US, vol. 15, no. 4, 1 December 2005 (2005-12-01), pages 284-297, XP002406791 ISSN: 1545-4576
- WILTON STEVE D ET AL: "Antisense oligonucleotide-induced exon skipping across the human dystrophin gene transcript" MOLECULAR THERAPY, NATURE PUBLISHING GROUP, GB, vol. 15, no. 7, 1 July 2007 (2007-07-01), pages 1288-1296, XP002544728 ISSN: 1525-0024
- AARTSMA-RUS ET AL: "Exploring the Frontiers of Therapeutic Exon Skipping for Duchenne Muscular Dystrophy by Double Targeting within One or Multiple Exons" MOLECULAR THERAPY, ACADEMIC PRESS, SAN DIEGO, CA, US, vol. 14, no. 3, 12 August 2006 (2006-08-12), pages 401-407, XP005844833 ISSN: 1525-0016

## Description

### Field

The invention relates to the field of genetics, more specifically human genetics. The invention in particular relates to modulation of splicing of the human Duchenne Muscular Dystrophy pre-mRNA.

### Background of the invention

Myopathies are disorders that result in functional impairment of muscles. Muscular dystrophy (MD) refers to genetic diseases that are characterized by progressive weakness and degeneration of skeletal muscles. Duchenne muscular dystrophy (DMD) and Becker muscular dystrophy (BMD) are the most common childhood forms of muscular dystrophy. They are recessive disorders and because the gene responsible for DMD and BMD resides on the X-chromosome, mutations mainly affect males with an incidence of about 1 in 3500 boys.

DMD and BMD are caused by genetic defects in the DMD gene encoding dystrophin, a muscle protein that is required for interactions between the cytoskeleton and the extracellular matrix to maintain muscle fiber stability during contraction. DMD is a severe, lethal neuromuscular disorder resulting in a dependency on wheelchair support before the age of 12 and DMD patients often die before the age of thirty due to respiratory- or heart failure. In contrast, BMD patients often remain ambulatory until later in life, and have near normal life expectancies. DMD mutations in the DMD gene are characterized by frame shifting insertions or deletions or nonsense point mutations, resulting in the absence of functional dystrophin. BMD mutations in general keep the reading frame intact, allowing synthesis of a partly functional dystrophin.

During the last decade, specific modification of splicing in order to restore the disrupted reading frame of the dystrophin transcript has emerged as a promising therapy for Duchenne muscular dystrophy (DMD) (van Ommen, van Deutekom, Aartsma-Rus, Curr Opin Mol Ther. 2008;10(2):140-9, Yokota, Duddy, Partidge, Acta Myol. 2007;26(3): 179-84, van Deutekom et al., N Engl J Med. 2007;357(26):2677-86). Using antisense oligonucleotides (AONs) interfering with splicing signals the skipping of specific exons can be induced in the DMD pre-mRNA, thus restoring the open reading frame and converting the severe DMD into a milder BMD phenotype (van Deutekom et al. Hum Mol Genet. 2001; 10: 1547-54; Aartsma-Rus et al., Hum Mol Genet 2003; 12(8):907-14.). *In vivo* proof-of-concept was first obtained in the *mdx* mouse model, which is dystrophin-deficient due to a nonsense mutation in exon 23. Intramuscular and intravenous injections of AONs targeting the mutated exon 23 restored dystrophin expression for at least three months (Lu et al. Nat Med. 2003; 8: 1009-14; Lu et al., Proc Natl Acad Sci U S A. 2005;102(1):198-203). This was accompanied by restoration of dystrophin-associated proteins at the fiber membrane as well as functional improvement of the treated muscle. *In vivo* skipping of human exons has also been achieved in the hDMD mouse model, which contains a complete copy of the human DMD gene integrated in chromosome 5 of the mouse (Bremmer-Bout et al. Molecular Therapy. 2004; 10: 232-40; 't Hoen et al. J Biol Chem. 2008; 283: 5899-907).

Recently, a first-in-man study was successfully completed where an AON inducing the skipping of exon 51 was injected into a small area of the tibialis anterior muscle of four DMD patients. Novel dystrophin expression was observed in the majority of muscle fibers in all four patients treated, and the AON was safe and well tolerated (van Deutekom et al. N Engl J Med. 2007; 357: 2677-86).

### Description of the invention

### Method

The invention is defined in the claims. In a first aspect, the present disclosure provides a method for inducing, and/or promoting skipping of at least one of exons 43, 46, 50- 53 of the DMD pre-mRNA in a patient, preferably in an isolated cell of a patient, the method comprising providing said cell and/or said patient with a molecule that binds to a continuous stretch of at least 8 nucleotides within said exon.

Accordingly, a method according to the invention is provided for inducing and/or promoting skipping of at least exon 52 of DMD pre-mRNA in an isolated cell of a patient, the method comprising providing said cell and/or said patient with an oligonucleotide that binds to a continuous stretch of at least 8 nucleotides within said exon.
It is to be understood that said method encompasses an *in vitro* or *ex vivo* method.

As defined herein a DMD pre-mRNA preferably means the pre-mRNA of a DMD gene of a DMD or BMD patient.

A patient is preferably intended to mean a patient having DMD or BMD as later defined herein or a patient susceptible to develop DMD or BMD due to his or her genetic background. In the case of a DMD patient, an oligonucleotide used will preferably correct one mutation as present in the DMD gene of said patient and therefore will preferably create a DMD protein that will look like a BMD protein: said protein will preferably be a functional dystrophin as later defined herein. In the case of a BMD patient, an oligonucleotide as used will preferably correct one mutation as present in the BMD gene of said patient and therefore will preferably create a dystrophin which will be more functional than the dystrophin which was originally present in said BMD patient.

Exon skipping refers to the induction in a cell of a mature mRNA that does not contain a particular exon that is normally present therein. Exon skipping is performed by providing a cell expressing the pre-mRNA of said mRNA with a molecule capable of interfering with essential sequences such as for example the splice donor of splice acceptor sequence that required for splicing of said exon, or a molecule that is capable of interfering with an exon inclusion signal that is required for recognition of a stretch of nucleotides as an exon to be included in the mRNA. The term pre-mRNA refers to a non-processed or partly processed precursor mRNA that is synthesized from a DNA template in the cell nucleus by transcription.

Within the context of the invention and disclosure, inducing and/or promoting skipping of an exon as indicated herein means that at least 1%, 10%, 20%, 30%, 40%,50%, 60%, 70%, 80%, 90% or more of the DMD mRNA in one or more (muscle) cells of a treated patient will not contain said exon. This is preferably assessed by PCR as described in the examples.

Preferably, a method of the invention or disclosure by inducing and/or promoting skipping of at least one of the following exons 43, 46, 50-53 of the DMD pre-mRNA in one or more (muscle) cells of a patient, provides said patient with a functional dystrophin protein and/or decreases the production of an aberrant dystrophin protein in said patient and/or increases the production of a functional dystrophin is said patient. Providing a patient with a functional dystrophin protein and/or decreasing the production of an aberrant dystrophin protein in said patient is typically applied in a DMD patient. Increasing the production of a functional dystrophin is typically applied in a BMD patient.
Therefore a preferred method of the invention or disclosure is a method, wherein a patient or one or more cells of said patient is provided with a functional dystrophin protein and/or wherein the production of an aberrant dystrophin protein in said patient is decreased and/or wherein the production of a functional dystrophin is increased in said patient, wherein the level of said aberrant or functional dystrophin is assessed by comparison to the level of said dystrophin in said patient at the onset of the method. Decreasing the production of an aberrant dystrophin may be assessed at the mRNA level and preferably means that 99%, 90%, 80%, 70%, 60%, 50%, 40%, 30%, 20%, 10%, 5% or less of the initial amount of aberrant dystrophin mRNA, is still detectable by RT PCR. An aberrant dystrophin mRNA or protein is also referred to herein as a non-functional dystrophin mRNA or protein. A non functional dystrophin protein is preferably a dystrophin protein which is not able to bind actin and/or members of the DGC protein complex. A non-functional dystrophin protein or dystrophin mRNA does typically not have, or does not encode a dystrophin protein with an intact C-terminus of the protein.
Increasing the production of a functional dystrophin in said patient or in a cell of said patient may be assessed at the mRNA level (by RT-PCR analysis) and preferably means that a detectable amount of a functional dystrophin mRNA is detectable by RT PCR. In another embodiment, 1%, 5%, 10%, 20%, 30%, 40%, 50%, 60%, 70%, 80%, 90% or more of the detectable dystrophin mRNA is a functional dystrophin mRNA.

Increasing the production of a functional dystrophin in said patient or in a cell of said patient may be assessed at the protein level (by immunofluorescence and western blot analyses) and preferably means that a detectable amount of a functional dystrophin protein is detectable by immunofluorescence or western blot analysis. In another embodiment, 1%, 5%, 10%, 20%, 30%, 40%, 50%, 60%, 70%, 80%, 90% or more of the detectable dystrophin protein is a functional dystrophin protein.

As defined herein, a functional dystrophin is preferably a wild type dystrophin corresponding to a protein having the amino acid sequence as identified in SEQ ID NO: 1. A functional dystrophin is preferably a dystrophin, which has an actin binding domain in its N terminal part (first 240 amino acids at the N terminus), a cystein-rich domain (amino acid 3361 till 3685) and a C terminal domain (last 325 amino acids at the C terminus) each of these domains being present in a wild type dystrophin as known to the skilled person. The amino acids indicated herein correspond to amino acids of the wild type dystrophin being represented by SEQ ID NO:1. In other words, a functional dystrophin is a dystrophin which exhibits at least to some extent an activity of a wild type dystrophin. "At least to some extent" preferably means at least 10%, 20%, 30%, 40%, 50%, 60%, 70%, 80%, 90%, 95% or 100% of a corresponding activity of a wild type functional dystrophin. In this context, an activity of a functional dystrophin is preferably binding to actin and to the dystrophin-associated glycoprotein complex (DGC) (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). Binding of dystrophin to actin and to the DGC complex may be visualized by either co-immunoprecipitation using total protein extracts or immunofluorescence analysis of cross-sections, from a muscle biopsy, as known to the skilled person.

Individuals or patients suffering from Duchenne muscular dystrophy typically have a mutation in the gene encoding dystrophin that prevent synthesis of the complete protein, i.e of a premature stop prevents the synthesis of the C-terminus. In Becker muscular dystrophy the DMD gene also comprises a mutation compared tot the wild type gene but the mutation does typically not induce a premature stop and the C-terminus is typically synthesized. As a result a functional dystrophin protein is synthesized that has at least the same activity in kind as the wild type protein, not although not necessarily the same amount of activity. The genome of a BMD individual typically encodes a dystrophin protein comprising the N terminal part (first 240 amino acids at the N terminus), a cystein-rich domain (amino acid 3361 till 3685) and a C terminal domain (last 325 amino acids at the C terminus) but its central rod shaped domain may be shorter than the one of a wild type dystrophin (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). Exon skipping for the treatment of DMD is typically directed to overcome a premature stop in the pre-mRNA by skipping an exon in the rod-shaped domain to correct the reading frame and allow synthesis of remainder of the dystrophin protein including the C-terminus, albeit that the protein is somewhat smaller as a result of a smaller rod domain. In a preferred embodiment, an individual having DMD and being treated by a method as defined herein will be provided a dystrophin which exhibits at least to some extent an activity of a wild type dystrophin. More preferably, if said individual is a Duchenne patient or is suspected to be a Duchenne patient, a functional dystrophin is a dystrophin of an individual having BMD: typically said dystrophin is able to interact with both actin and the DGC, but its central rod shaped domain may be shorter than the one of a wild type dystrophin (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). The central rod-shaped domain of wild type dystrophin comprises 24 spectrin-like repeats (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). For example, a central rod-shaped domain of a dystrophin as provided herein may comprise 5 to 23, 10 to 22 or 12 to 18 spectrin-like repeats as long as it can bind to actin and to DGC.

A method of the invention or disclosure may alleviate one or more characteristics of a myogenic or muscle cell of a patient or alleviate one or more symptoms of a DMD patient having a deletion including but not limited to exons 44, 44-46, 44-47, 44-48, 44-49, 44-51, 44-53 (correctable by exon 43 skipping), 19-45, 21-45, 43-45, 45, 47-54, 47-56 (correctable by exon 46 skipping), 51, 51-53, 51-55, 51-57 (correctable by exon 50 skipping), 13-50, 19-50, 29-50, 43-50, 45-50, 47-50, 48-50, 49-50, 50, 52 (correctable by exon 51 skipping), exons 8-51, 51, 53, 53-55, 53-57, 53-59, 53-60, (correctable by exon 52 skipping) and exons 10-52, 42-52, 43-52, 45-52, 47-52, 48-52, 49-52, 50-52, 52 (correctable by exon 53 skipping) in the DMD gene, occurring in a total of 68% of all DMD patients with a deletion (Aartsma-Rus et al., Hum. Mut. 2009).
Alternatively, a method of the invention or disclosure may improve one or more characteristics of a muscle cell of a patient or alleviate one or more symptoms of a DMD patient having small mutations in, or single exon duplications of exon 43, 46, 50-53 in the DMD gene, occurring in a total of 36% of all DMD patients with a deletion (Aartsma-Rus et al, Hum. Mut. 2009)

Furthermore, for some patients the simultaneous skipping of one of more exons in addition to exon 43, exon 46 and/or exon 50-53 is required to restore the open reading frame, including patients with specific deletions, small (point) mutations, or double or multiple exon duplications, such as (but not limited to) a deletion of exons 44-50 requiring the co-skipping of exons 43 and 51, with a deletion of exons 46-50 requiring the co-skipping of exons 45 and 51, with a deletion of exons 44-52 requiring the co-skipping of exons 43 and 53, with a deletion of exons 46-52 requiring the co-skipping of exons 45 and 53, with a deletion of exons 51-54 requiring the co-skipping of exons 50 and 55, with a deletion of exons 53-54 requiring the co-skipping of exons 52 and 55, with a deletion of exons 53-56 requiring the co-skipping of exons 52 and 57, with a nonsense mutation in exon 43 or exon 44 requiring the co-skipping of exon 43 and 44, with a nonsense mutation in exon 45 or exon 46 requiring the co-skipping of exon 45 and 46, with a nonsense mutation in exon 50 or exon 51 requiring the co-skipping of exon 50 and 51, with a nonsense mutation in exon 51 or exon 52 requiring the co-skipping of exon 51 and 52, with a nonsense mutation in exon 52 or exon 53 requiring the co-skipping of exon 52 and 53, or with a double or multiple exon duplication involving exons 43, 46, 50, 51, 52, and/or 53.

In a preferred method of the disclosure, the skipping of exon 43 is induced, or the skipping of exon 46 is induced, or the skipping of exon 50 is induced or the skipping of exon 51 is induced or the skipping of exon 52 is induced or the skipping of exon 53 is induced. An induction of the skipping of two of these exons is also encompassed by a method of the disclosure. For example, preferably skipping of exons 50 and 51, or 52 and 53, or 43 and 51, or 43 and 53, or 51 and 52. Depending on the type and the identity (the specific exons involved) of mutation identified in a patient, the skilled person will know which combination of exons needs to be skipped in said patient.

In a preferred method, one or more symptom(s) of a DMD or a BMD patient is/are alleviated and/or one or more characteristic(s) of one or more muscle cells from a DMD or a BMD patient is/are improved. Such symptoms or characteristics may be assessed at the cellular, tissue level or on the patient self.

An alleviation of one or more characteristics may be assessed by any of the following assays on a myogenic cell or muscle cell from a patient: reduced calcium uptake by muscle cells, decreased collagen synthesis, altered morphology, altered lipid biosynthesis, decreased oxidative stress, and/or improved muscle fiber function, integrity, and/or survival. These parameters are usually assessed using immunofluorescence and/or histochemical analyses of cross sections of muscle biopsies.
The improvement of muscle fiber function, integrity and/or survival may be assessed using at least one of the following assays: a detectable decrease of creatine kinase in blood, a detectable decrease of necrosis of muscle fibers in a biopsy cross-section of a muscle suspected to be dystrophic, and/or a detectable increase of the homogeneity of the diameter of muscle fibers in a biopsy cross-section of a muscle suspected to be dystrophic. Each of these assays is known to the skilled person.

Creatine kinase may be detected in blood as described in Hodgetts et al (Hodgetts S., et al, (2006), Neuromuscular Disorders, 16: 591-602.2006). A detectable decrease in creatine kinase may mean a decrease of 5%, 10%, 20%, 30%, 40%, 50%, 60%, 70%, 80%, 90% or more compared to the concentration of creatine kinase in a same DMD or BMD patient before treatment.

A detectable decrease of necrosis of muscle fibers is preferably assessed in a muscle biopsy, more preferably as described in Hodgetts et al (Hodgetts S., et al, (2006), Neuromuscular Disorders, 16: 591-602.2006) using biopsy cross-sections. A detectable decrease of necrosis may be a decrease of 5%, 10%, 20%, 30%, 40%, 50%, 60%, 70%, 80%, 90% or more of the area wherein necrosis has been identified using biopsy cross-sections. The decrease is measured by comparison to the necrosis as assessed in a same DMD or BMD patient before treatment.

A detectable increase of the homogeneity of the diameter of a muscle fiber is preferably assessed in a muscle biopsy cross-section, more preferably as described in Hodgetts et al (Hodgetts S., et al, (2006), Neuromuscular Disorders, 16: 591-602.2006). The increase is measured by comparison to the homogeneity of the diameter of a muscle fiber in a same DMD or BMD patient before treatment
An alleviation of one or more symptoms may be assessed by any of the following assays on the patient self: prolongation of time to loss of walking, improvement of muscle strength, improvement of the ability to lift weight, improvement of the time taken to rise from the floor, improvement in the nine-meter walking time, improvement in the time taken for four-stairs climbing, improvement of the leg function grade, improvement of the pulmonary function, improvement of cardiac function, improvement of the quality of life. Each of these assays is known to the skilled person. As an example, the publication of Manzur at al (Manzur AY et al, (2008), Glucocorticoid corticosteroids for Duchenne muscular dystrophy (review), Wiley publishers, The Cochrane collaboration.) gives an extensive explanation of each of these assays. For each of these assays, as soon as a detectable improvement or prolongation of a parameter measured in an assay has been found, it will preferably mean that one or more symptoms of Duchenne Muscular Dystrophy or Becker Muscular Dystrophy has been alleviated in an individual using a method of the invention. Detectable improvement or prolongation is preferably a statistically significant improvement or prolongation as described in Hodgetts et al (Hodgetts S., et al, (2006), Neuromuscular Disorders, 16: 591-602.2006). Alternatively, the alleviation of one or more symptom(s) of Duchenne Muscular Dystrophy or Becker Muscular Dystrophy may be assessed by measuring an improvement of a muscle fiber function, integrity and/or survival as later defined herein.

A treatment in a method according to the invention may have a duration of at least one week, at least one month, at least several months, at least one year, at least 2, 3, 4, 5, 6 years or more.
Each molecule or oligonucleotide or equivalent thereof as defined herein for use according to the invention may be suitable for direct administration to a cell, tissue and/or an organ *in vivo* of individuals affected by or at risk of developing DMD or BMD, and may be administered directly *in vivo, ex vivo* or *in vitro.* The frequency of administration of a molecule or an oligonucleotide or a composition of the invention may depend on several parameters such as the age of the patient, the mutation of the patient, the number of molecules (dose), the formulation of said molecule. The frequency may be ranged between at least once in a two weeks, or three weeks or four weeks or five weeks or a longer time period.

A molecule or oligonucleotide or equivalent thereof can be delivered as is to a cell. When administering said molecule, oligonucleotide or equivalent thereof to an individual, it is preferred that it is dissolved in a solution that is compatible with the delivery method. For intravenous, subcutaneous, intramuscular, intrathecal and/or intraventricular administration it is preferred that the solution is a physiological salt solution. Particularly preferred for a method of the invention is the use of an excipient that will further enhance delivery of said molecule, oligonucleotide or functional equivalent thereof as defined herein, to a cell and into a cell, preferably a muscle cell. Preferred excipient are defined in the section entitled "pharmaceutical composition".

In a preferred method of the invention, an additional antisense oligonucleotide is used which is able to induce and/or promote skipping of another exon of the DMD pre-mRNA of a patient. Preferably, the second exon is selected from: exon 51, 53, 55, 57, 59 or 69 of the DMD pre-mRNA of a patient. Molecules which can be used are depicted in any one of Table 1 to 7. This way, inclusion of two or more exons of a DMD pre-mRNA in mRNA produced from this pre-mRNA is prevented. This embodiment is further referred to as double- or multi-exon skipping (Aartsma-Rus A, Janson AA, Kaman WE, et al. Antisense-induced multiexon skipping for Duchenne muscular dystrophy makes more sense. Am J Hum Genet 2004;74(1):83-92, Aartsma-Rus A, Kaman WE, Weij R, den Dunnen JT, van Ommen GJ, van Deutekom JC. Exploring the frontiers of therapeutic exon skipping for Duchenne muscular dystrophy by double targeting within one or multiple exons. Mol Ther 2006;14(3):401-7). In most cases double-exon skipping results in the exclusion of only the two targeted exons from the DMD pre-mRNA. However, in other cases it was found that the targeted exons and the entire region in between said exons in said pre-mRNA were not present in the produced mRNA even when other exons (intervening exons) were present in such region. This multi-skipping was notably so for the combination of oligonucleotides derived from the DMD gene, wherein one oligonucleotide for exon 45 and one oligonucleotide for exon 51 was added to a cell transcribing the DMD gene. Such a setup resulted in mRNA being produced that did not contain exons 45 to 51. Apparently, the structure of the pre-mRNA in the presence of the mentioned oligonucleotides was such that the splicing machinery was stimulated to connect exons 44 and 52 to each other.
It is possible to specifically promote the skipping of also the intervening exons by providing a linkage between the two complementary oligonucleotides. Hence, in one embodiment stretches of nucleotides complementary to at least two dystrophin exons are separated by a linking moiety. The at least two stretches of nucleotides are thus linked in this embodiment so as to form a single molecule.

In case, more than one compounds or molecules are used in a method of the invention or disclosure, said compounds can be administered to an individual in any order. In one embodiment, said compounds are administered simultaneously (meaning that said compounds are administered within 10 hours, preferably within one hour). This is however not necessary. In another embodiment, said compounds are administered sequentially.

### Molecule

In a second aspect, there is provided a molecule for use in a method as described in the previous section entitled "Method". A molecule as defined herein is preferably an oligonucleotide or antisense oligonucleotide (AON).

It was found by the present investigators that any of exon 43, 46, 50-53 is specifically skipped at a high frequency using a molecule that preferably binds to a continuous stretch of at least 8 nucleotides within said exon. Although this effect can be associated with a higher binding affinity of said molecule, compared to a molecule that binds to a continuous stretch of less than 8 nucleotides, there could be other intracellular parameters involved that favor thermodynamic, kinetic, or structural characteristics of the hybrid duplex. In a preferred embodiment, a molecule that binds to a continuous stretch of at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37,38, 39, 40, 41, 42, 43,44, 45, 46, 47, 48, 49, 50 nucleotides within said exon is used.

In a preferred embodiment, a molecule or an oligonucleotide of the disclosure which comprises a sequence that is complementary to a part of any of exon 43, 46, 50, 51 or 53 of DMD pre-mRNA, or a molecule or an oligonucleotide of the invention which comprises a sequence that is complementary to a part of exon 52 of DMD pre-mRNA, is such that the complementary part is at least 50% of the length of the oligonucleotide of the invention, more preferably at least 60%, even more preferably at least 70%, even more preferably at least 80%, even more preferably at least 90% or even more preferably at least 95%, or even more preferably 98% and most preferably up to 100%. "A part of said exon" preferably means a stretch of at least 8 nucleotides. In a most preferred embodiment, an oligonucleotide of the invention or disclosure consists of a sequence that is complementary to part of said exon DMD pre-mRNA as defined herein. For example, an oligonucleotide may comprise a sequence that is complementary to part of said exon DMD pre-mRNA as defined herein and additional flanking sequences. In a more preferred embodiment, the length of said complementary part of said oligonucleotide is of at least 8, 9, 10, 11, 12, 13, 14, 15, 16, 17, 18, 19, 20, 21, 22, 23, 24, 25, 26, 27 , 28 , 29, 30, 31, 32, 33, 34, 35, 36, 37, 38, 39, 40, 41, 42, 43, 44, 45, 46, 47, 48, 49, 50 nucleotides. Preferably, additional flanking sequences are used to modify the binding of a protein to said molecule or oligonucleotide, or to modify a thermodynamic property of the oligonucleotide, more preferably to modify target RNA binding affinity.

A preferred molecule to be used in a method of the disclosure binds or is complementary to a continuous stretch of at least 8 nucleotides within one of the following nucleotide sequences selected from:
5'-AGAUAGUCUACAACAAAGCUCAGGUCGGAUUGACAUUAUUCAUAGCAAGAAGACAGCAGCAUUGCAAAGUGCAACGCCUGUGG-3' (SEQ ID NO: 2) for skipping of exon 43;
5'-UUAUGGUUGGAGGAAGCAGAUAACAUUGCUAGUAUCCCACUUGAACCUGGAAAAGAGCAGCAACUAAAAGAAAAGC-3' (SEQ ID NO: 3) for skipping of exon 46;
5'-GGCGGTAAACCGUUUACUUCAAGAGCUGAGGGCAAAGCAGCCUGACCUAGC UCCUGGACUGACCACUAUUGG-3' (SEQ ID NO: 4) for skipping of exon 50;
5'-CUCCUACUCAGACUGUUACUCUGGUGACACAACCUGUGGUUACUAAGGAAACUGCCAUCUCCAAACUAGAAAUGCCAUCUUCCUUGAUGUUGGAGGUAC-3' (SEQ ID NO: 5) for skipping of exon 51; and
5'-AAAUGUUAAAGGAUUCAACACAAUGGCUGGAAGCUAAGGAAGAAGCUGAGCAGGUCUUAGGACAGGCCAGAG-3' (SEQ ID NO:7) for skipping of exon 53.

A preferred molecule to be used in a method of the invention binds or is complementary to a continuous stretch of at least 8 nucleotides within the following nucleotide sequence:
5'-AUGCAGGAUUUGGAACAGAGGCGUCCCCAGUUGGAAGAACUCAUUACCGCUGCCCAAAAUUUGAAAAACAAGACCAGCAAUCAAGAGGCU-3' (SEQ ID NO:6) for skipping of exon 52.

Of the numerous molecules that theoretically can be prepared to bind to the continuous nucleotide stretches as defined by SEQ ID NO 2-7 within one of said exons, the invention and disclosure provide distinct molecules that can be used in a method for efficiently skipping of at least one of exon 43, exon 46 and/or exon 50- 53. Although the skipping effect can be addressed to the relatively high density of putative SR protein binding sites within said stretches, there could be other parameters involved that favor uptake of the molecule or other, intracellular parameters such as thermodynamic, kinetic, or structural characteristics of the hybrid duplex.

It was found that a molecule that binds to a continuous stretch comprised within or consisting of any of SEQ ID NO 2-7 results in highly efficient skipping of exon 43, exon 46 and/or exon 50- 53 respectively in a cell and/or in a patient provided with this molecule. Therefore, in a preferred embodiment, a method is provided wherein a molecule binds to a continuous stretch of at least 8, 10, 12, 15, 18, 20, 25, 30, 35, 40, 45, 50 nucleotides within SEQ ID NO 2-7.

In a preferred embodiment for inducing and/or promoting the skipping of any of exon 43, exon 46 and/or exon 50- 53, the invention or disclosure provide a molecule comprising or consisting of an antisense nucleotide sequence selected from the antisense nucleotide sequences depicted in any of Tables 1 to 6. A molecule of the disclosure preferably comprises or consist of the antisense nucleotide sequence of SEQ ID NO 16, SEQ ID NO 65, SEQ ID NO 70, SEQ ID NO 91, SEQ ID NO 110, SEQ ID NO 117, SEQ ID NO 127, SEQ ID NO 165, SEQ ID NO 166, SEQ ID NO 167, SEQ ID NO 246, SEQ ID NO 299, SEQ ID NO:357.

A preferred molecule of the disclosure comprises a nucleotide-based or nucleotide or an antisense oligonucleotide sequence of between 8 and 50 nucleotides or bases, more preferred between 10 and 50 nucleotides, more preferred between 20 and 40 nucleotides, more preferred between 20 and 30 nucleotides, such as 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, 45 nucleotides, 46 nucleotides, 47 nucleotides, 48 nucleotides, 49 nucleotides or 50 nucleotides.
A most preferred molecule of the invention or disclosure comprises a nucleotide-based sequence of 25 nucleotides.

Furthermore, none of the indicated sequences is derived from conserved parts of splice-junction sites. Therefore, said molecule is not likely to mediate differential splicing of other exons from the DMD pre-mRNA or exons from other genes.

In one embodiment, a molecule of the invention or disclosure is a compound molecule that binds to the specified sequence, or a protein such as an RNA-binding protein or a non-natural zinc-finger protein that has been modified to be able to bind to the corresponding nucleotide sequence on a DMD pre-RNA molecule. Methods for screening compound molecules that bind specific nucleotide sequences are, for example, disclosed in PCT/NL01/00697 and US Patent 6,875,736. Methods for designing RNA-binding Zinc-finger proteins that bind specific nucleotide sequences are disclosed by Friesen and Darby, Nature Structural Biology 5: 543- 546 (1998).

A preferred molecule of the disclosure binds to at least part of the sequence of SEQ ID NO 2: 5'-AGAUAGUCUACAACAAAGCUCAGGUCGGAUUGACAUUAUUCAUAGCAAGAAGACAGCAGCAUUGCAAAGUGCAACGCCUGUGG-3' which is present in exon 43 of the DMD gene. More preferably, the disclosure provides a molecule comprising or consisting of the antisense nucleotide sequence of SEQ ID NO 8 to SEQ ID NO 69.
In an even more preferred embodiment, the disclosure provides a molecule comprising or consisting of the antisense nucleotide sequence of SEQ ID NO 16 and/or SEQ ID NO 65.
In a most preferred embodiment, the disclosure provides a molecule comprising or consisting of the antisense nucleotide sequence of SEQ ID NO 65. It was found that this molecule is very efficient in modulating splicing of exon 43 of the DMD pre-mRNA in a muscle cell and/or in a patient.

Another preferred molecule of the disclosure binds to at least part of the sequence of SEQ ID NO 3: 5'-UUAUGGUUGGAGGAAGCAGAUAACAUUGCUAGUAUCCCACUUGAACCUGGAAAAGAGCAGCAACUAAAAGAAAAGC-3' which is present in exon 46 of the DMD gene. More preferably, the disclosure provides a molecule comprising or consisting of the antisense nucleotide sequence of SEQ ID NO 70 to SEQ ID NO 122.
In an even more preferred embodiment, the disclosure provides a molecule comprising or consisting of the antisense nucleotide sequence of SEQ ID NO 70, SEQ ID NO 91, SEQ ID NO 110, and/or SEQ ID NO117.
In a most preferred embodiment, the disclosure provides a molecule comprising or consisting of the antisense nucleotide sequence of SEQ ID NO 117. It was found that this molecule is very efficient in modulating splicing of exon 46 of the DMD pre-mRNA in a muscle cell or in a patient.

Another preferred molecule of the disclosure binds to at least part of the sequence of SEQ ID NO 4: 5'-GGCGGTAAACCGUUUACUUCAAGAGCUGAGGGCAAAGCAGCCUGACCUAGCUCCUGGACUGACCACUAUUGG-3' which is present in exon 50 of the DMD gene. More preferably, the disclosure provides a molecule comprising or consisting of the antisense nucleotide sequence of SEQ ID NO 123 to SEQ ID NO 167 and/or SEQ ID NO 529 to SEQ ID NO 535.
In an even more preferred embodiment, the disclosure provides a molecule comprising or consisting of the antisense nucleotide sequence of SEQ ID NO 127, or SEQ ID NO 165, or SEQ ID NO 166 and/or SEQ ID NO 167.
In a most preferred embodiment, the disclosure provides a molecule comprising or consisting of the antisense nucleotide sequence of SEQ ID NO 127. It was found that this molecule is very efficient in modulating splicing of exon 50 of the DMD pre-mRNA in a muscle cell and/or in a patient.

Another preferred molecule of the disclosure binds to at least part of the sequence of SEQ ID NO 5:5'-CUCCUACUCAGACUGUUACUCUGGUGACACAACCUGUGGUUACUAAGGAAACUGCCAUCUCCAAACUAGAAAUGCCAUCUUCCUUGAUGUUGGAGGUAC-3' which is present in exon 51 of the DMD gene. More preferably, the disclosure provides a molecule comprising or consisting of the antisense nucleotide sequence of SEQ ID NO 168 to SEQ ID NO 241.

Another preferred molecule of the invention binds to at least part of the sequence of SEQ ID NO 6:
5'-AUGCAGGAUUUGGAACAGAGGCGUCCCCAGUUGGAAGAACUCAUUACCGCUGCCCAAAAUUUGAAAAACAAGACCAGCAAUCAAGAGGCU-3' which is present in exon 52 of the DMD gene. More preferably, the invention or disclosure provide a molecule comprising or consisting of the antisense nucleotide sequence of SEQ ID NO 242 to SEQ ID NO 310. In an even more preferred embodiment, the invention or disclosure provide a molecule comprising or consisting of the antisense nucleotide sequence of SEQ ID NO 246 and/or SEQ ID NO 299. In a most preferred embodiment, the disclosure provides a molecule comprising or consisting of the antisense nucleotide sequence of SEQ ID NO 299. It was found that this molecule is very efficient in modulating splicing of exon 52 of the DMD pre-mRNA in a muscle cell and/or in a patient. The invention provides a molecule comprising or consisting of the antisense nucleotide sequence of SEQ ID NO: 287.

Another preferred molecule of the disclosure binds to at least part of the sequence of SEQ ID NO 7: 5'-AAAUGUUAAAGGAUUCAACACAAUGGCUGGAAGCUAAGGAAGAAGCUGAGCAGGUCUUAGGACAGGCCAGAG-3' which is present in exon 53 of the DMD gene. More preferably, the disclosure provides a molecule comprising or consisting of the antisense nucleotide sequence of SEQ ID NO 311 to SEQ ID NO 358.
In a most preferred embodiment, the disclosure provides a molecule comprising or consisting of the antisense nucleotide sequence of SEQ ID NO 357. It was found that this molecule is very efficient in modulating splicing of exon 53 of the DMD pre-mRNA in a muscle cell and/or in a patient.

A nucleotide sequence of a molecule of the invention and disclosure may contain RNA residues, or one or more DNA residues, and/or one or more nucleotide analogues or equivalents, as will be further detailed herein below.

It is preferred that a molecule of the invention comprises one or more residues that are modified to increase nuclease resistance, and/or to increase the affinity of the antisense nucleotide for the target sequence. Therefore, in a preferred embodiment, the antisense nucleotide sequence comprises at least one nucleotide analogue or equivalent, wherein a nucleotide analogue or equivalent is defined as a residue having a modified base, and/or a modified backbone, and/or a non-natural internucleoside linkage, or a combination of these modifications.

In a preferred embodiment, the nucleotide analogue or equivalent comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones. Phosphorodiamidate morpholino oligomers are modified backbone oligonucleotides that have previously been investigated as antisense agents. Morpholino oligonucleotides have an uncharged backbone in which the deoxyribose sugar of DNA is replaced by a six membered ring and the phosphodiester linkage is replaced by a phosphorodiamidate linkage. Morpholino oligonucleotides are resistant to enzymatic degradation and appear to function as antisense agents by arresting translation or interfering with pre-mRNA splicing rather than by activating RNase H. Morpholino oligonucleotides have been successfully delivered to tissue culture cells by methods that physically disrupt the cell membrane, and one study comparing several of these methods found that scrape loading was the most efficient method of delivery; however, because the morpholino backbone is uncharged, cationic lipids are not effective mediators of morpholino oligonucleotide uptake in cells. A recent report demonstrated triplex formation by a morpholino oligonucleotide and, because of the non-ionic backbone, these studies showed that the morpholino oligonucleotide was capable of triplex formation in the absence of magnesium.

It is further preferred that that the linkage between the residues in a backbone do not include a phosphorus atom, such as a linkage that is formed by short chain alkyl or cycloalkyl internucleoside linkages, mixed heteroatom and alkyl or cycloalkyl internucleoside linkages, or one or more short chain heteroatomic or heterocyclic internucleoside linkages.

A preferred nucleotide analogue or equivalent comprises a Peptide Nucleic Acid (PNA), having a modified polyamide backbone (Nielsen, et al. (1991) Science 254, 1497-1500). PNA-based molecules are true mimics of DNA molecules in terms of base-pair recognition. The backbone of the PNA is composed of *N*-(2-aminoethyl)-glycine units linked by peptide bonds, wherein the nucleobases are linked to the backbone by methylene carbonyl bonds. An alternative backbone comprises a one-carbon extended pyrrolidine PNA monomer (Govindaraju and Kumar (2005) Chem. Commun, 495-497). Since the backbone of a PNA molecule contains no charged phosphate groups, PNA-RNA hybrids are usually more stable than RNA-RNA or RNA-DNA hybrids, respectively (Egholm et al (1993) Nature 365, 566-568).

A further preferred backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.

In yet a further embodiment, a nucleotide analogue or equivalent of the invention comprises a substitution of one of the non-bridging oxygens in the phosphodiester linkage. This modification slightly destabilizes base-pairing but adds significant resistance to nuclease degradation. A preferred nucleotide analogue or equivalent comprises phosphorothioate, chiral phosphorothioate, phosphorodithioate, phosphotriester, aminoalkylphosphotriester, H-phosphonate, methyl and other alkyl phosphonate including 3'-alkylene phosphonate, 5'-alkylene phosphonate and chiral phosphonate, phosphinate, phosphoramidate including 3'-amino phosphoramidate and aminoalkylphosphoramidate, thionophosphoramidate, thionoalkylphosphonate, thionoalkylphosphotriester, selenophosphate or boranophosphate.

A further preferred nucleotide analogue or equivalent of the invention comprises one or more sugar moieties that are mono- or disubstituted at the 2', 3' and/or 5' position such as a -OH; -F; substituted or unsubstituted, linear or branched lower (C1-C10) alkyl, alkenyl, alkynyl, alkaryl, allyl, aryl, or aralkyl, that may be interrupted by one or more heteroatoms; O-, S-, or N-alkyl; O-, S-, or N-alkenyl; O-, S-or N-alkynyl; O-, S-, or N-allyl; O-alkyl-O-alkyl, -methoxy, -aminopropoxy; -aminoxy; methoxyethoxy; - dimethylaminooxyethoxy; and -dimethylaminoethoxyethoxy. The sugar moiety can be a pyranose or derivative thereof, or a deoxypyranose or derivative thereof, preferably a ribose or a derivative thereof, or a deoxyribose or a derivative thereof. Such preferred derivatized sugar moieties comprise Locked Nucleic Acid (LNA), in which the 2'-carbon atom is linked to the 3' or 4' carbon atom of the sugar ring thereby forming a bicyclic sugar moiety. A preferred LNA comprises 2'-*O*,4'-C-ethylene-bridged nucleic acid (Morita et al. 2001. Nucleic Acid Res Supplement No.1: 241-242). These substitutions render the nucleotide analogue or equivalent RNase H and nuclease resistant and increase the affinity for the target RNA.

It is understood by a skilled person that it is not necessary for all positions in an antisense oligonucleotide to be modified uniformly. In addition, more than one of the aforementioned analogues or equivalents may be incorporated in a single antisense oligonucleotide or even at a single position within an antisense oligonucleotide. In certain embodiments, an antisense oligonucleotide of the invention has at least two different types of analogues or equivalents.

A preferred antisense oligonucleotide according to the invention comprises a 2'-*O* alkyl phosphorothioate antisense oligonucleotide, such as 2'-*O*-methyl modified ribose (RNA), 2'-*O*-ethyl modified ribose, 2'-*O*-propyl modified ribose, and/or substituted derivatives of these modifications such as halogenated derivatives.

A most preferred antisense oligonucleotide according to the invention comprises of 2'-*O*-methyl phosphorothioate ribose.

A functional equivalent of a molecule of the invention or disclosure may be defined as an oligonucleotide as defined herein wherein an activity of said functional equivalent is retained to at least some extent. Preferably, an activity of said functional equivalent is inducing exon 43, 46, 50, 51, 52, or 53 skipping and providing a functional dystrophin protein. Said activity of said functional equivalent is therefore preferably assessed by detection of exon 43, 46, 50, 51, 52, or 53 skipping and by quantifying the amount of functional dystrophin protein. A functional dystrophin is herein preferably defined as being a dystrophin able to bind actin and members of the DGC protein complex. The assessment of said activity of an oligonucleotide is preferably done by RT-PCR or by immunofluorescence or Western blot analyses. Said activity is preferably retained to at least some extent when it represents at least 50%, or at least 60%, or at least 70% or at least 80% or at least 90% or at least 95% or more of corresponding activity of said oligonucleotide the functional equivalent derives from. Throughout this application, when the word oligonucleotide is used it may be replaced by a functional equivalent thereof as defined herein.

It will be understood by a skilled person that distinct antisense oligonucleotides can be combined for efficiently skipping any of exon 43, exon 46, exon 50, exon 51, exon 52 and/or exon 53 of the human DMD pre-mRNA. It is encompassed by the present invention or disclosure to use one, two, three, four, five or more oligonucleotides for skipping one of said exons (i.e. exon, 43, 46, 50, 51, 52, or 53). It is also encompassed to use at least two oligonucleotides for skipping at least two, of said exons. Preferably two of said exons are skipped. More preferably, these two exons are:
- 43 and 51, or
- 43 and 53, or
- 50 and 51, or
- 51 and 52, or
- 52 and 53.
The skilled person will know which combination of exons is preferred to be skipped depending on the type, the number and the location of the mutation present in a DMD or BMD patient.

An antisense oligonucleotide can be linked to a moiety that enhances uptake of the antisense oligonucleotide in cells, preferably muscle cells. Examples of such moieties are cholesterols, carbohydrates, vitamins, biotin, lipids, phospholipids, cell-penetrating peptides including but not limited to antennapedia, TAT, transportan and positively charged amino acids such as oligoarginine, poly-arginine, oligolysine or polylysine, antigen-binding domains such as provided by an antibody, a Fab fragment of an antibody, or a single chain antigen binding domain such as a cameloid single domain antigen-binding domain.

A preferred antisense oligonucleotide comprises a peptide-linked PMO.

A preferred antisense oligonucleotide comprising one or more nucleotide analogs or equivalents of the invention modulates splicing in one or more muscle cells, including heart muscle cells, upon systemic delivery. In this respect, systemic delivery of an antisense oligonucleotide comprising a specific nucleotide analog or equivalent might result in targeting a subset of muscle cells, while an antisense oligonucleotide comprising a distinct nucleotide analog or equivalent might result in targeting of a different subset of muscle cells. Therefore, in one embodiment it is preferred to use a combination of antisense oligonucleotides comprising different nucleotide analogs or equivalents for inducing skipping of exon 43, 46, 50, 51, 52, or 53 of the human DMD pre-mRNA.

A cell can be provided with a molecule capable of interfering with essential sequences that result in highly efficient skipping of exon 43, exon 46, exon 50, exon 51, exon 53 (according to disclosure) or exon 52 (according to invention) of the human DMD pre-mRNA by plasmid-derived antisense oligonucleotide expression or viral expression provided by adenovirus- or adeno-associated virus-based vectors. In a preferred embodiment, there is provided a viral-based expression vector comprising an expression cassette that drives expression of a molecule as identified herein. Expression is preferably driven by a polymerase III promoter, such as a U1, a U6, or a U7 RNA promoter. A muscle or myogenic cell can be provided with a plasmid for antisense oligonucleotide expression by providing the plasmid in an aqueous solution. Alternatively, a plasmid can be provided by transfection using known transfection agentia such as, for example, LipofectAMINE™ 2000 (Invitrogen) or polyethyleneimine (PEI; ExGen500 (MBI Fermentas)), or derivatives thereof.

One preferred antisense oligonucleotide expression system is an adenovirus associated virus (AAV)-based vector. Single chain and double chain AAV-based vectors have been developed that can be used for prolonged expression of small antisense nucleotide sequences for highly efficient skipping of exon 43, 46, 50, 51, 52 or 53 of the DMD pre-mRNA.

A preferred AAV-based vector comprises an expression cassette that is driven by a polymerase III-promoter (Pol III). A preferred Pol III promoter is, for example, a U1, a U6, or a U7 RNA promoter.

The invention therefore also provides a viral-based vector, comprising a Pol III-promoter driven expression cassette for expression of an antisense sequence of the invention for inducing skipping of exon 52 of the human DMD pre-mRNA. A viral-based vector according to the disclosure comprises a Pol III-promoter driven expression cassette for expression of one or more antisense sequences of the disclosure for inducing skipping of exon 43, exon 46, exon 50, exon 51 or exon 53 of the human DMD pre-mRNA.

### Pharmaceutical composition

If required, a molecule or a vector expressing an antisense oligonucleotide of the invention can be incorporated into a pharmaceutically active mixture or composition by adding a pharmaceutically acceptable carrier.

Therefore, in a further aspect, the invention provides a composition, preferably a pharmaceutical composition comprising a molecule comprising an antisense oligonucleotide according to the invention, and/or a viral-based vector expressing the antisense sequence(s) according to the invention and a pharmaceutically acceptable carrier.

A preferred pharmaceutical composition comprises a molecule as defined herein and/or a vector as defined herein, and a pharmaceutical acceptable carrier or excipient, optionally combined with a molecule and/or a vector as defined herein which is able to induce skipping of exon 51, 53, 55, 57, 59 or69 of the DMD pre-mRNA. Preferred molecules able to induce skipping of any of these exon are identified in any one of Tables 1 to 7.

Preferred excipients include excipients capable of forming complexes, vesicles and/or liposomes that deliver such a molecule as defined herein, preferably an oligonucleotide complexed or trapped in a vesicle or liposome through a cell membrane. Many of these excipients are known in the art. Suitable excipients comprise polyethylenimine and derivatives, or similar cationic polymers, including polypropyleneimine or polyethylenimine copolymers (PECs) and derivatives, ExGen 500, synthetic amphiphils (SAINT-18), lipofectinTM, DOTAP and/or viral capsid proteins that are capable of self assembly into particles that can deliver such molecule, preferably an oligonucleotide as defined herein to a cell, preferably a muscle cell. Such excipients have been shown to efficiently deliver (oligonucleotide such as antisense) nucleic acids to a wide variety of cultured cells, including muscle cells. Their high transfection potential is combined with an excepted low to moderate toxicity in terms of overall cell survival. The ease of structural modification can be used to allow further modifications and the analysis of their further (*in vivo*) nucleic acid transfer characteristics and toxicity.

Lipofectin represents an example of a liposomal transfection agent. It consists of two lipid components, a cationic lipid N-[1-(2,3dioleoyloxy)propyl]-N,N,N-trimethylammonium chloride (DOTMA) (cp. DOTAP which is the methylsulfate salt) and a neutral lipid dioleoylphosphatidylethanolamine (DOPE). The neutral component mediates the intracellular release. Another group of delivery systems are polymeric nanoparticles.

Polycations such like diethylaminoethylaminoethyl (DEAE)-dextran, which are well known as DNA transfection reagent can be combined with butylcyanoacrylate (PBCA) and hexylcyanoacrylate (PHCA) to formulate cationic nanoparticles that can deliver a molecule or a compound as defined herein, preferably an oligonucleotide across cell membranes into cells.

In addition to these common nanoparticle materials, the cationic peptide protamine offers an alternative approach to formulate a compound as defined herein, preferably an oligonucleotide as colloids. This colloidal nanoparticle system can form so called proticles, which can be prepared by a simple self-assembly process to package and mediate intracellular release of a compound as defined herein, preferably an oligonucleotide. The skilled person may select and adapt any of the above or other commercially available alternative excipients and delivery systems to package and deliver a compound as defined herein, preferably an oligonucleotide for use in the current invention to deliver said compound for the treatment of Duchenne Muscular Dystrophy or Becker Muscular Dystrophy in humans.

In addition, a compound as defined herein, preferably an oligonucleotide could be covalently or non-covalently linked to a targeting ligand specifically designed to facilitate the uptake in to the cell, cytoplasm and/or its nucleus. Such ligand could comprise (i) a compound (including but not limited to peptide(-like) structures) recognising cell, tissue or organ specific elements facilitating cellular uptake and/or (ii) a chemical compound able to facilitate the uptake in to cells and/or the intracellular release of an a compound as defined herein, preferably an oligonucleotide from vesicles, e.g. endosomes or lysosomes.

Therefore, in a preferred embodiment, a compound as defined herein, preferably an oligonucleotide are formulated in a medicament which is provided with at least an excipient and/or a targeting ligand for delivery and/or a delivery device of said compound to a cell and/or enhancing its intracellular delivery. Accordingly, the invention also encompasses a pharmaceutically acceptable composition comprising a compound as defined herein, preferably an oligonucleotide and further comprising at least one excipient and/or a targeting ligand for delivery and/or a delivery device of said compound to a cell and/or enhancing its intracellular delivery.
It is to be understood that a molecule or compound or oligonucleotide may not be formulated in one single composition or preparation. Depending on their identity, the skilled person will know which type of formulation is the most appropriate for each compound.

In a preferred embodiment, an in vitro concentration of a molecule or an oligonucleotide as defined herein, which is ranged between 0.1 nM and 1 □M is used. More preferably, the concentration used is ranged between 0.3 to 400 nM, even more preferably between 1 to 200 nM. A molecule or an oligonucleotide as defined herein may be used at a dose which is ranged between 0.1 and 20 mg/kg, preferably 0.5 and 10 mg/kg. If several molecules or oligonucleotides are used, these concentrations may refer to the total concentration of oligonucleotides or the concentration of each oligonucleotide added. The ranges of concentration of oligonucleotide(s) as given above are preferred concentrations for *in vitro* or *ex vivo* uses. The skilled person will understand that depending on the oligonucleotide(s) used, the target cell to be treated, the gene target and its expression levels, the medium used and the transfection and incubation conditions, the concentration of oligonucleotide(s) used may further vary and may need to be optimised any further.

More preferably, a compound preferably an oligonucleotide to be used in the invention or disclosure to prevent, treat DMD or BMD are synthetically produced and administered directly to a cell, a tissue, an organ and/or patients in formulated form in a pharmaceutically acceptable composition or preparation. The delivery of a pharmaceutical composition to the subject is preferably carried out by one or more parenteral injections, e.g. intravenous and/or subcutaneous and/or intramuscular and/or intrathecal and/or intraventricular administrations, preferably injections, at one or at multiple sites in the human body.

A preferred oligonucleotide as defined herein optionally comprising one or more nucleotide analogs or equivalents of the invention or disclosure modulates splicing in one or more muscle cells, including heart muscle cells, upon systemic delivery. In this respect, systemic delivery of an oligonucleotide comprising a specific nucleotide analog or equivalent might result in targeting a subset of muscle cells, while an oligonucleotide comprising a distinct nucleotide analog or equivalent might result in targeting of a different subset of muscle cells.

In this respect, systemic delivery of an oligonucleotide comprising a specific nucleotide analog or equivalent might result in targeting a subset of muscle cells, while an oligonucleotide comprising a distinct nucleotide analog or equivalent might result in targeting a different subset of muscle cells. Therefore, in this embodiment, it is preferred to use a combination of oligonucleotides comprising different nucleotide analogs or equivalents for modulating splicing of the DMD mRNA in at least one type of muscle cells.

In a preferred embodiment of the invention, there is provided a molecule or a viral-based vector for use as a medicament, preferably for modulating splicing of the DMD pre-mRNA, more preferably for promoting or inducing skipping of exon 52 as identified herein. In a preferred embodiment of the disclosure, there is provided a molecule or a viral-based vector for use as a medicament, preferably for modulating splicing of the DMD pre-mRNA, more preferably for promoting or inducing skipping of any of exon 43, 46, 50, 51 and 53 as identified herein.

### Use

In yet a further aspect, the invention provides the use of an antisense oligonucleotide or molecule according to the invention, and/or a viral-based vector that expresses anantisense sequence according to the invention and/or a pharmaceutical composition, for modulating splicing of the DMD pre-mRNA. The splicing is preferably modulated in a human myogenic cell or muscle cell *in vitro.* More preferred is that splicing is modulated in a human muscle cell *in vivo.* Accordingly,
the invention further relates to the use of the molecule as defined herein and/or the vector as defined herein and/or or the pharmaceutical composition as defined herein for modulating splicing of the DMD pre-mRNA or for the preparation of a medicament for the treatment of a DMD or BMD patient.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a molecule or a viral-based vector or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".
Each embodiment as identified herein may be combined together unless otherwise indicated.

The following examples are offered for illustrative purposes only, and are not intended to limit the scope of the present invention in any way.

### Examples

### Examples 1-4

### Materials and methods

AON design was based on (partly) overlapping open secondary structures of the target exon RNA as predicted by the m-fold program, on (partly) overlapping putative SR-protein binding sites as predicted by the ESE-finder software. AONs were synthesized by Prosensa Therapeutics B.V. (Leiden, Netherlands), and contain 2'-*O*-methyl RNA and full-length phosphorothioate (PS) backbones.

### Tissue culturing, transfection and RT-PCR analysis

Myotube cultures derived from a healthy individual ("human control") (examples 1,3, and 4; exon 43, 50, 52 skipping) or a DMD patient carrying an exon 45 deletion (example 2; exon 46 skipping) were processed as described previously (Aartsma-Rus et al., Neuromuscul. Disord. 2002; 12: S71-77 and Hum Mol Genet 2003; 12(8): 907-14). For the screening of AONs, myotube cultures were transfected with 50 nM and 150 nM (example 2), 200nM and 500 nM (example 4) or 500nM only (examples 1 and 3) of each AON. Transfection reagent UNIFectylin (Prosensa Therapeutics BV, Netherlands) was used, with 2 µl UNIFectylin per µg AON. Exon skipping efficiencies were determined by nested RT-PCR analysis using primers in the exons flanking the targeted exons (43, 46, 50, 51, 52, or 53). PCR fragments were isolated from agarose gels for sequence verification. For quantification, the PCR products were analyzed using the DNA 1000 LabChips Kit on the Agilent 2100 bioanalyzer (Agilent Technologies, USA).

### Results

### DMD exon 43 skipping.

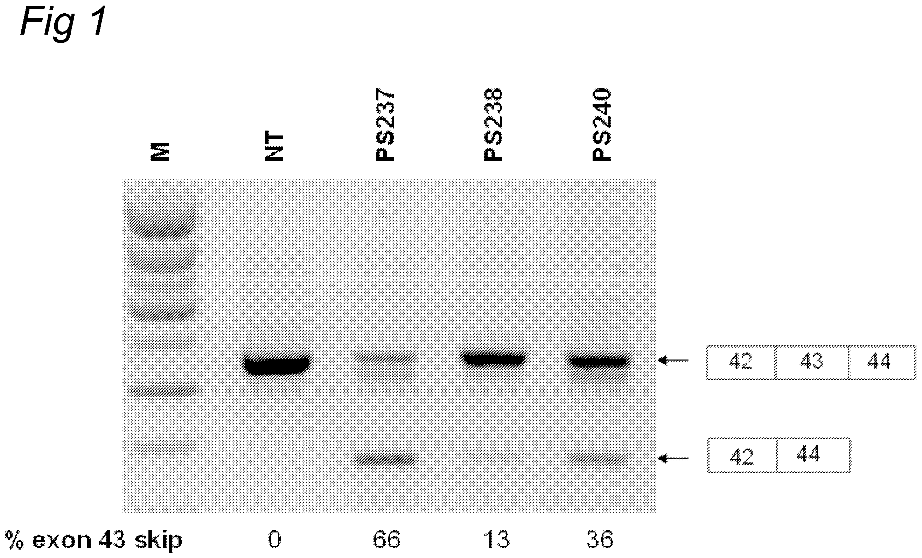

A series of AONs targeting sequences within exon 43 were designed and transfected in healthy control myotube cultures. Subsequent RT-PCR and sequence analysis of isolated RNA demonstrated that almost all AONs targeting a continuous nucleotide stretch within exon 43 herein defined as SEQ ID NO 2, was indeed capable of inducing exon 43 skipping. PS237 (SEQ ID NO: 65) reproducibly induced highest levels of exon 43 skipping (up to 66%) at 500nM, as shown in Figure 1. For comparison, also PS238 and PS240 are shown, inducing exon 43 skipping levels up to 13% and 36% respectively (Figure 1). The precise skipping of exon 43 was confirmed by sequence analysis of the novel smaller transcript fragments. No exon 43 skipping was observed in non-treated cells (NT).

### DMD exon 46 skipping.

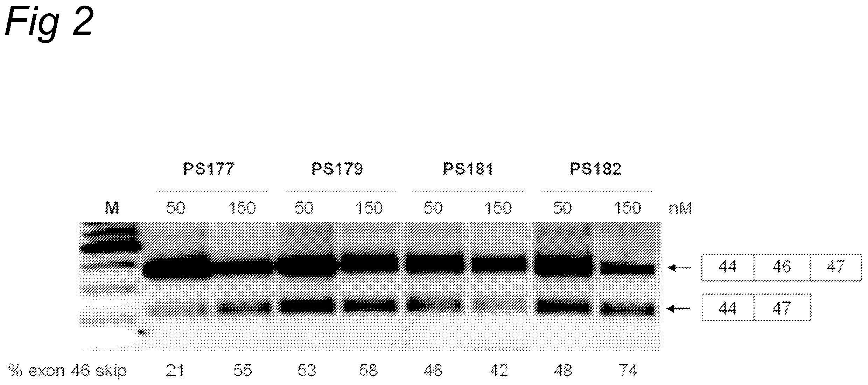

A series of AONs targeting sequences within exon 46 were designed and transfected in myotube cultures derived from a DMD patient carrying an exon 45 deletion in the DMD gene. For patients with such mutation antisense-induced exon 46 skipping would induce the synthesis of a novel, BMD-like dystrophin protein that may indeed alleviate one or more symptoms of the disease. Subsequent RT-PCR and sequence analysis of isolated RNA demonstrated that almost all AONs targeting a continuous nucleotide stretch within exon 46 herein defined as SEQ ID NO 3, was indeed capable of inducing exon 46 skipping, even at relatively low AON concentrations of 50 nM. PS182 (SEQ ID NO: 117) reproducibly induced highest levels of exon 46 skipping (up to 50% at 50 nM and 74% at 150 nM), as shown in Figure 2. For comparison, also PS177, PS179, and PS181 are shown, inducing exon 46 skipping levels up to 55%, 58% and 42% respectively at 150 nM (Figure 2). The precise skipping of exon 46 was confirmed by sequence analysis of the novel smaller transcript fragments. No exon 46 skipping was observed in non-treated cells (NT).

### DMD exon 50 skipping.

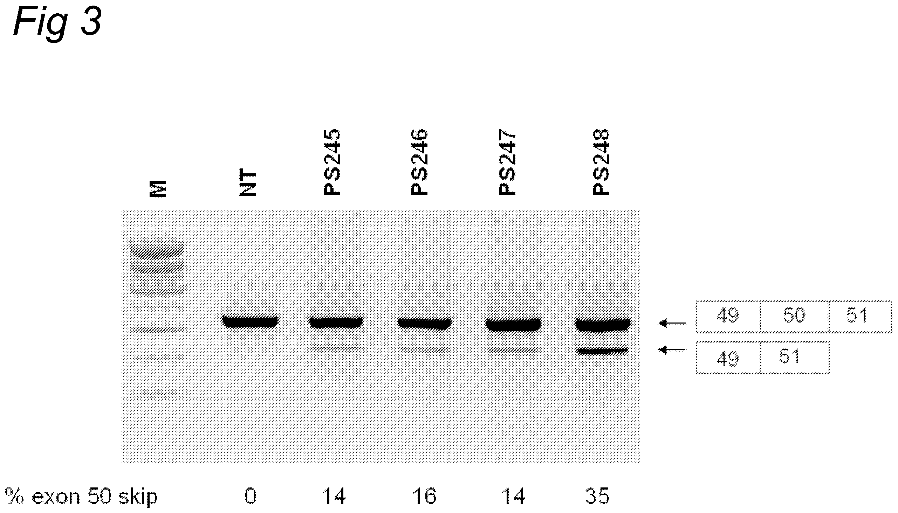

A series of AONs targeting sequences within exon 50 were designed and transfected in healthy control myotube cultures. Subsequent RT-PCR and sequence analysis of isolated RNA demonstrated that almost all AONs targeting a continuous nucleotide stretch within exon 50 herein defined as SEQ ID NO 4, was indeed capable of inducing exon 50 skipping. PS248 (SEQ ID NO: 127) reproducibly induced highest levels of exon 50 skipping (up to 35% at 500 nM), as shown in Figure 3. For comparison, also PS245, PS246, and PS247 are shown, inducing exon 50 skipping levels up to 14-16% at 500 nM (Figure 3). The precise skipping of exon 50 was confirmed by sequence analysis of the novel smaller transcript fragments. No exon 50 skipping was observed in non-treated cells (NT).

### DMD exon 51 skipping.

A series of AONs targeting sequences within exon 51 were designed and transfected in healthy control myotube cultures. Subsequent RT-PCR and sequence analysis of isolated RNA demonstrated that almost all AONs targeting a continuous nucleotide stretch within exon 51 herein defined as SEQ ID NO 5, was indeed capable of inducing exon 51 skipping. The AON with SEQ ID NO 180 reproducibly induced highest levels of exon 51 skipping (not shown).

### DMD exon 52 skipping.

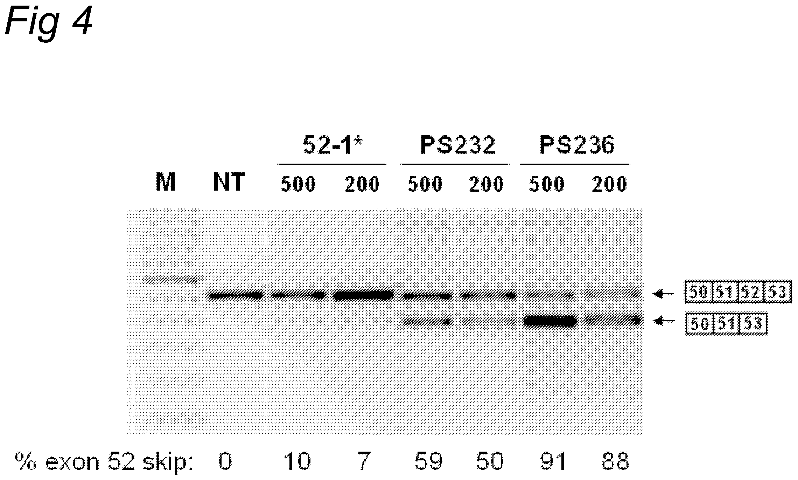

A series of AONs targeting sequences within exon 52 were designed and transfected in healthy control myotube cultures. Subsequent RT-PCR and sequence analysis of isolated RNA demonstrated that almost all AONs targeting a continuous nucleotide stretch within exon 52 herein defined as SEQ ID NO 6, was indeed capable of inducing exon 52 skipping. PS236 (SEQ ID NO: 299) reproducibly induced highest levels of exon 52 skipping (up to 88% at 200 nM and 91% at 500 nM), as shown in Figure 4. For comparison, also PS232 and AON 52-1 (previously published by Aartsma-Rus et al. Oligonucleotides 2005) are shown, inducing exon 52 skipping at levels up to 59% and 10% respectively when applied at 500 nM (Figure 4). The precise skipping of exon 52 was confirmed by sequence analysis of the novel smaller transcript fragments. No exon 52 skipping was observed in non-treated cells (NT).

### DMD exon 53 skipping.

A series of AONs targeting sequences within exon 53 were designed and transfected in healthy control myotube cultures. Subsequent RT-PCR and sequence analysis of isolated RNA demonstrated that almost all AONs targeting a continuous nucleotide stretch within exon 53 herein defined as SEQ ID NO 7, was indeed capable of inducing exon 53 skipping. The AON with SEQ ID NO 328 reproducibly induced highest levels of exon 53 skipping (not shown).

### Sequence listing:

### DMD gene amino acid sequence

**SEQ ID NO 1:** 
MLWWEEVEDCYEREDVQKKTFTKWVNAQFSKFGKQHIENLFSDLQDGRRLLDLLEGLTGQKL
PKEKGSTRVHALNNVNKALRVLQNNNVDLVNIGSTDIVDGNHKLTLGLIWNIILHWQVKNVMK
NIMAGLQQTNSEKILLSWVRQSTRNYPQVNVINFTTSWSDGLALNALIHSHRPDLFDWNSVVCQ
QSATQRLEHAFNIARYQLGIEKLLDPEDVDTTYPDKKSILMYITSLFQVLPQQVSIEAIQEVEMLP
RPPKVTKEEHFQLHHQMHYSQQITVSLAQGYERTSSPKPRFKSYAYTQAAYVTTSDPTRSPFPSQ
HLEAPEDKSFGSSLMESEVNLDRYQTALEEVLSWLLSAEDTLQAQGEISNDVEVVKDQFHTHEG
YMMDLTAHQGRVGNILQLGSKLIGTGKLSEDEETEVQEQMNLLNSRWECLRVASMEKQSNLH
RVLMDLQNQKLKELNDWLTKTEERTRKMEEEPLGPDLEDLKRQVQQHKVLQEDLEQEQVRVN
SLTHMVVVVDESSGDHATAALEEQLKVLGDRWANICRWTEDRWVLLQDILLKWQRLTEEQCL
FSAWLSEKEDAVNKIHTTGFKDQNEMLSSLQKLAVLKADLEKKKQSMGKLYSLKQDLLSTLKN
KSVTQKTEAWLDNFARCWDNLVQKLEKSTAQISQAVTTTQPSLTQTTVMETVTTVTTREQILV
KHAQEELPPPPPQKKRQITVDSEIRKRLDVDITELHSWITRSEAVLQSPEFAIFRKEGNFSDLKEK
VNAIEREKAEKFRKLQDASRSAQALVEQMVNEGVNADSIKQASEQLNSRWIEFCQLLSERLNW
LEYQNNIIAFYNQLQQLEQMTTTAENWLKIQPTTPSEPTAIKSQLKICKDEVNRLSGLQPQIERLK
IQSIALKEKGQGPMFLDADFVAFTNHFKQVFSDVQAREKELQTIFDTLPPMRYQETMSAIRTWV
QQSETKLSIPQLSVTDYEIMEQRLGELQALQSSLQEQQSGLYYLSTTVKEMSKKAPSEISRKYQS
EFEEIEGRWKKLSSQLVEHCQKLEEQMNKLRKIQNHIQTLKKWMAEVDVFLKEEWPALGDSEI
LKKQLKQCRLLVSDIQTIQPSLNSVNEGGQKIKNEAEPEFASRLETELKELNTQWDHMCQQVYA
RKEALKGGLEKTVSLQKDLSEMHEWMTQAEEEYLERDFEYKTPDELQKAVEEMKRAKEEAQQ
KEAKVKLLTESVNSVIAQAPPVAQEALKKELETLTTNYQWLCTRLNGKCKTLEEVWACWHELL
SYLEKANKWLNEVEFKLKTTENIPGGAEEISEVLDSLENLMRHSEDNPNQIRILAQTLTDGGVM
DELINEELETFNSRWRELHEEAVRRQKLLEQSIQSAQETEKSLHLIQESLTFIDKQLAAYIADKVD
AAQMPQEAQKIQSDLTSHEISLEEMKKHNQGKEAAQRVLSQIDVAQKKLQDVSMKFRLFQKPA
NFEQRLQESKMILDEVKMHLPALETKSVEQEVVQSQLNHCVNLYKSLSEVKSEVEMVIKTGRQI
VQKKQTENPKELDERVTALKLHYNELGAKVTERKQQLEKCLKLSRKMRKEMNVLTEWLAAT
DMELTKRSAVEGMPSNLDSEVAWGKATQKEIEKQKVHLKSITEVGEALKTVLGKKETLVEDKL
SLLNSNWIAVTSRAEEWLNLLLEYQKHMETFDQNVDHITKWIIQADTLLDESEKKKPQQKEDVL
KRLKAELNDIRPKVDSTRDQAANLMANRGDHCRKLVEPQISELNHRFAAISHRIKTGKASIPLKE
LEQFNSDIQKLLEPLEAEIQQGVNLKEEDFNKDMNEDNEGTVKELLQRGDNLQQRITDERKREEI
KIKQQLLQTKHNALKDLRSQRRKKALEISHQWYQYKRQADDLLKCLDDIEKKLASLPEPRDER
KIKEIDRELQKKKEELNAVRRQAEGLSEDGAAMAVEPTQIQLSKRWREIESKFAQFRRLNFAQIH
TVREETMMVMTEDMPLEISYVPSTYLTEITHVSQALLEVEQLLNAPDLCAKDFEDLFKQEESLK
NIKDSLQQSSGRIDIIHSKKTAALQSATPVERVKLQEALSQLDFQWEKVNKMYKDRQGRFDRSV
EKWRRFHYDIKIFNQWLTEAEQFLRKTQIPENWEHAKYKWYLKELQDGIGQRQTVVRTLNATG
 
EEIIQQSSKTDASILQEKLGSLNLRWQEVCKQLSDRKKRLEEQKNILSEFQRDLNEFVLWLEEAD
NIASIPLEPGKEQQLKEKLEQVKLLVEELPLRQGILKQLNETGGPVLVSAPISPEEQDKLENKLKQ
TNLQWIKVSRALPEKQGEIEAQIKDLGQLEKKLEDLEEQLNHLLLWLSPIRNQLEIYNQPNQEGP
FDVQETEIAVQAKQPDVEEILSKGQHLYKEKPATQPVKRKLEDLSSEWKAVNRLLQELRAKQP
DLAPGLTTIGASPTQTVTLVTQPVVTKETAISKLEMPSSLMLEVPALADFNRAWTELTDWLSLL
DQVIKSQRVMVGDLEDINEMIIKQKATMQDLEQRRPQLEELITAAQNLKNKTSNQEARTIITDRI
ERIQNQWDEVQEHLQNRRQQLNEMLKDSTQWLEAKEEAEQVLGQARAKLESWKEGPYTVDAI
QKKITETKQLAKDLRQWQTNVDVANDLALKLLRDYSADDTRKVHMITENINASWRSIHKRVSE
REAALEETHRLLQQFPLDLEKFLAWLTEAETTANVLQDATRKERLLEDSKGVKELMKQWQDL
QGEIEAHTDVYHNLDENSQKILRSLEGSDDAVLLQRRLDNMNFKWSELRKKSLNIRSHLEASSD
QWKRLHLSLQELLVWLQLKDDELSRQAPIGGDFPAVQKQNDVHRAFKRELKTKEPVIMSTLET
VRIFLTEQPLEGLEKLYQEPRELPPEERAQNVTRLLRKQAEEVNTEWEKLNLHSADWQRKIDET
LERLQELQEATDELDLKLRQAEVIKGSWQPVGDLLIDSLQDHLEKVKALRGEIAPLKENVSHVN
DLARQLTTLGIQLSPYNLSTLEDLNTRWKLLQVAVEDRVRQLHEAHRDFGPASQHFLSTSVQGP
WERAISPNKVPYYINHETQTTCWDHPKMTELYQSLADLNNVRFSAYRTAMKLRRLQKALCLDL
LSLSAACDALDQHNLKQNDQPMDILQIINCLTTIYDRLEQEHNNLVNVPLCVDMCLNWLLNVY
DTGRTGRIRVLSFKTGIISLCKAHLEDKYRYLFKQVASSTGFCDQRRLGLLLHDSIQIPRQLGEVA
SFGGSNIEPSVRSCFQFANNKPEIEAALFLDWMRLEPQSMVWLPVLHRVAAAETAKHQAKCNIC
KECPIIGFRYRSLKHFNYDICQSCFFSGRVAKGHKMHYPMVEYCTPTTSGEDVRDFAKVLKNKF
RTKRYFAKHPRMGYLPVQTVLEGDNMETPVTLINFWPVDSAPASSPQLSHDDTHSRIEHYASRL
AEMENSNGSYLNDSISPNESIDDEHLLIQHYCQSLNQDSPLSQPRSPAQILISLESEERGELERILAD
LEEENRNLQAEYDRLKQQHEHKGLSPLPSPPEMMPTSPQSPRDAELIAEAKLLRQHKGRLEARM
QILEDHNKQLESQLHRLRQLLEQPQAEAKVNGTTVSSPSTSLQRSDSSQPMLLRVVGSQTSDSM
GEEDLLSPPQDTSTGLEEVMEQLNNSFPSSRGRNTPGKPMREDTM
**SEQ ID NO 2 (exon 43):** 
AGAUAGUCUACAACAAAGCUCAGGUCGGAUUGACAUUAUUCAUAGCAAGAAGACAGCAG
CAUUGCAAAGUGCAACGCCUGUGG
**SEQ ID NO 3 (exon 46):** 
UUAUGGUUGGAGGAAGCAGAUAACAUUGCUAGUAUCCCACUUGAACCUGGAAAAGAGCA
GCAACUAAAAGAAAAGC
**SEQ ID NO 4 (exon 50):** 
’GGCGGTAAACCGUUUACUUCAAGAGCUGAGGGCAAAGCAGCCUG ACCUAGC
UCCUGGACUGACCACUAUUGG
**SEQ ID NO 5 (exon 51):** 
CUCCUACUCAGACUGUUACUCUGGUGACACAACCUGUGGUUACUAAGGAAACUGCCAUC
UCCAAACUAGAAAUGCCAUCUUCCUUGAUGUUGGAGGUAC
**SEQ ID NO 6 (exon 52):** 
AUGCAGGAUUUGGAACAGAGGCGUCCCCAGUUGGAAGAACUCAUUACCGCUGCCCAAAA
UUUGAAAAA CAAGACCAGCAAUCAAGAGGCU
**SEQ ID NO 7 (exon 53):**

AAAUGUUAAAGGAUUCAACACAAUGGCUGGAAGCUAAGGAAGAAGCUGAGCAGGUCUUA
GGACAGGCCAGAG

**Table 1: oligonucleotides for skipping DMD Gene Exon 43**

| | | | |
|---|---|---|---|
| SEQ ID NO 8 | CCACAGGCGUUGCACUUUGCAAUGC | SEQ ID NO 39 | UCUUCUUGCUAUGAAUAAUGUCAAU |
| SEQ ID NO 9 | CACAGGCGUUGCACUUUGCAAUGCU | SEQ ID NO 40 | CUUCUUGCUAUGAAUAAUGUCAAUC |
| SEQ ID NO 10 | ACAGGCGUUGCACUUUGCAAUGCUG | SEQ ID NO 41 | UUCUUGCUAUGAAUAAUGUCAAUCC |
| SEQ ID NO 11 | CAGGCGUUGCACUUUGCAAUGCUGC | SEQ ID NO 42 | UCUUGCUAUGAAUAAUGUCAAUCCG |
| SEQ ID NO 12 | AGGCGUUGCACUUUGCAAUGCUGCU | SEQ ID NO 43 | CUUGCUAUGAAUAAUGUCAAUCCGA |
| SEQ ID NO 13 | GGCGUUGCACUUUGCAAUGCUGCUG | SEQ ID NO 44 | UUGCUAUGAAUAAUGUCAAUCCGAC |
| SEQ ID NO 14 | GCGUUGCACUUUGCAAUGCUGCUGU | SEQ ID NO 45 | UGCUAUGAAUAAUGUCAAUCCGACC |
| SEQ ID NO 15 | CGUUGCACUUUGCAAUGCUGCUGUC | SEQ ID NO 46 | GCUAUGAAUAAUGUCAAUCCGACCU |
| SEQ ID NO 16 **PS240** | CGUUGCACUUUGCAAUGCUGCUG | SEQ ID NO 47 | CUAUGAAUAAUGUCAAUCCGACCUG |
| SEQ ID NO 17 | GUUGCACUUUGCAAUGCUGCUGUCU | SEQ ID NO 48 | UAUGAAUAAUGUCAAUCCGACCUGA |
| SEQ ID NO 18 | UUGCACUUUGCAAUGCUGCUGUCUU | SEQ ID NO 49 | AUGAAUAAUGUCAAUCCGACCUGAG |
| SEQ ID NO 19 | UGCACUUUGCAAUGCUGCUGUCUUC | SEQ ID NO 50 | UGAAUAAUGUCAAUCCGACCUGAGC |
| SEQ ID NO 20 | GCACUUUGCAAUGCUGCUGUCUUCU | SEQ ID NO 51 | GAAUAAUGUCAAUCCGACCUGAGCU |
| SEQ ID NO 21 | CACUUUGCAAUGCUGCUGUCUUCUU | SEQ ID NO 52 | AAUAAUGUCAAUCCGACCUGAGCUU |
| SEQ ID NO 22 | ACUUUGCAAUGCUGCUGUCUUCUUG | SEQ ID NO 53 | AUAAUGUCAAUCCGACCUGAGCUUU |
| SEQ ID NO 23 | CUUUGCAAUGCUGCUGUCUUCUUGC | SEQ ID NO 54 | UAAUGUCAAUCCGACCUGAGCUUUG |
| SEQ ID NO 24 | UUUGCAAUGCUGCUGUCUUCUUGCU | SEQ ID NO 55 | AAUGUCAAUCCGACCUGAGCUUUGU |
| SEQ ID NO 25 | UUGCAAUGCUGCUGUCUUCUUGCUA | SEQ ID NO 56 | AUGUCAAUCCGACCUGAGCUUUGUU |
| SEQ ID NO 26 | UGCAAUGCUGCUGUCUUCUUGCUAU | SEQ ID NO 57 | UGUCAAUCCGACCUGAGCUUUGUUG |
| SEQ ID NO 27 | GCAAUGCUGCUGUCUUCUUGCUAUG | SEQ ID NO 58 | GUCAAUCCGACCUGAGCUUUGUUGU |
| SEQ ID NO 28 | CAAUGCUGCUGUCUUCUUGCUAUGA | SEQ ID NO 59 | UCAAUCCGACCUGAGCUUUGUUGUA |
| SEQ ID NO 29 | AAUGCUGCUGUCUUCUUGCUAUGAA | SEQ ID NO 60 | CAAUCCGACCUGAGCUUUGUUGUAG |
| SEQ ID NO 30 | AUGCUGCUGUCUUCUUGCUAUGAAU | SEQ ID NO 61 | AAUCCGACCUGAGCUUUGUUGUAGA |
| SEQ ID NO 31 | UGCUGCUGUCUUCUUGCUAUGAAUA | SEQ ID NO 62 | AUCCGACCUGAGCUUUGUUGUAGAC |
| SEQ ID NO 32 | GCUGCUGUCUUCUUGCUAUGAAUAA | SEQ ID NO 63 | UCCGACCUGAGCUUUGUUGUAGACU |
| SEQ ID NO 33 | CUGCUGUCUUCUUGCUAUGAAUAAU | SEQ ID NO 64 | CCGACCUGAGCUUUGUUGUAGACUA |
| SEQ ID NO 34 | UGCUGUCUUCUUGCUAUGAAUAAU G | SEQ ID NO 65 **PS237** | CGACCUGAGCUUUGUUGUAG |
| SEQ ID NO 35 | GCUGUCUUCUUGCUAUGAAUAAUG U | SEQ ID NO 66 **PS238** | CGACCUGAGCUUUGUUGUAGACUAU |
| SEQ ID NO 36 | CUGUCUUCUUGCUAUGAAUAAUGUC | SEQ ID NO 67 | GACCUGAGCUUUGUUGUAGACUAUC |
| SEQ ID NO 37 | UGUCUUCUUGCUAUGAAUAAUGUC A | SEQ ID NO 68 | ACCUGAGCUUUGUUGUAGACUAUCA |
| SEQ ID NO 38 | GUCUUCUUGCUAUGAAUAAUGUCA A | SEQ ID NO 69 | CCUGA GCUUU GUUGU AGACU AUC |

**Table 2: oligonucleotides for skipping DMD Gene Exon 46**

| | | | |
|---|---|---|---|
| SEQ ID NO 70 **PS179** | GCUUUUCUUUUAGUUGCUGCUCUUU | SEQ ID NO 97 | CCAGGUUCAAGUGGGAUACUAGCAA |
| SEQ ID NO 71 | CUUUUCUUUUAGUUGCUGCUCUUUU | SEQ ID NO 98 | CAGGUUCAAGUGGGAUACUAGCAAU |
| SEQ ID NO 72 | UUUUCUUUUAGUUGCUGCUCUUUUC | SEQ ID NO 99 | AGGUUCAAGUGGGAUACUAGCAAUG |
| SEQ ID NO 73 | UUUCUUUUAGUUGCUGCUCUUUUCC | SEQ ID NO 100 | GGUUCAAGUGGGAUACUAGCAAUGU |
| SEQ ID NO 74 | UUCUUUUAGUUGCUGCUCUUUUCCA | SEQ ID NO 101 | GUUCAAGUGGGAUACUAGCAAUGUU |
| SEQ ID NO 75 | UCUUUUAGUUGCUGCUCUUUUCCAG | SEQ ID NO 102 | UUCAAGUGGGAUACUAGCAAUGUUA |
| SEQ ID NO 76 | CUUUUAGUUGCUGCUCUUUUCCAGG | SEQ ID NO 103 | UCAAGUGGGAUACUAGCAAUGUUAU |
| SEQ ID NO 77 | UUUUAGUUGCUGCUCUUUUCCAGGU | SEQ ID NO 104 | CAAGUGGGAUACUAGCAAUGUUAUC |
| SEQ ID NO 78 | UUUAGUUGCUGCUCUUUUCCAGGUU | SEQ ID NO 105 | AAGUGGGAUACUAGCAAUGUUAUCU |
| SEQ ID NO 79 | UUAGUUGCUGCUCUUUUCCAGGUUC | SEQ ID NO 106 | AGUGGGAUACUAGCAAUGUUAUCUG |
| SEQ ID NO 80 | UAGUUGCUGCUCUUUUCCAGGUUCA | SEQ ID NO 107 | GUGGGAUACUAGCAAUGUUAUCUGC |
| SEQ ID NO 81 | AGUUGCUGCUCUUUUCCAGGUUCAA | SEQ ID NO 108 | UGGGAUACUAGCAAUGUUAUCUGCU |
| SEQ ID NO 82 | GUUGCUGCUCUUUUCCAGGUUCAAG | SEQ ID NO 109 | GGGAUACUAGCAAUGUUAUCUGCUU |
| SEQ ID NO 83 | UUGCUGCUCUUUUCCAGGUUCAAGU | SEQ ID NO 110 **PS181** | GGAUACUAGCAAUGUUAUCUGCUUC |
| SEQ ID NO 84 | UGCUGCUCUUUUCCAGGUUCAAGUG | SEQ ID NO 111 | GAUACUAGCAAUGUUAUCUGCUUCC |
| SEQ ID NO 85 | GCUGCUCUUUUCCAGGUUCAAGUGG | SEQ ID NO 112 | AUACUAGCAAUGUUAUCUGCUUCCU |
| SEQ ID NO 86 | CUGCUCUUUUCCAGGUUCAAGUGGG | SEQ ID NO 113 | UACUAGCAAUGUUAUCUGCUUCCUC |
| SEQ ID NO 87 | UGCUCUUUUCCAGGUUCAAGUGGGA | SEQ ID NO 114 | ACUAGCAAUGUUAUCUGCUUCCUCC |
| SEQ ID NO 88 | GCUCUUUUCCAGGUUCAAGUGGGAC | SEQ ID NO 115 | CUAGCAAUGUUAUCUGCUUCCUCCA |
| SEQ ID NO 89 | CUCUUUUCCAGGUUCAAGUGGGAUA | SEQ ID NO 116 | UAGCAAUGUUAUCUGCUUCCUCCAA |
| SEQ ID NO 90 | UCUUUUCCAGGUUCAAGUGGGAUAC | SEQ ID NO 117 **PS182** | AGCAAUGUUAUCUGCUUCCUCCAAC |
| SEQ ID NO 91 **PS177** | UCUUUUCCAGGUUCAAGUGG | SEQ ID NO 118 | GCAAUGUUAUCUGCUUCCUCCAACC |
| SEQ ID NO 92 | CUUUUCCAGGUUCAAGUGGGAUACU | SEQ ID NO 119 | CAAUGUUAUCUGCUUCCUCCAACCA |
| SEQ ID NO 93 | UUUUCCAGGUUCAAGUGGGAUACU A | SEQ ID NO 120 | AAUGUUAUCUGCUUCCUCCAACCAU |
| SEQ ID NO 94 | UUUCCAGGUUCAAGUGGGAUACUA G | SEQ ID NO 121 | AUGUUAUCUGCUUCCUCCAACCAUA |
| SEQ ID NO 95 | UUCCAGGUUCAAGUGGGAUACUAGC | SEQ ID NO 122 | UGUUAUCUGCUUCCUCCAACCAUAA |
| SEQ ID NO 96 | UCCAGGUUCAAGUGGGAUACUAGCA | | |

**Table 3: oligonucleotides for skipping DMD Gene Exon 50**

| | | | |
|---|---|---|---|
| SEQ ID NO 123 | CCAAUAGUGGUCAGUCCAGGAGCUA | SEQ ID NO 146 | CUAGGUCAGGCUGCUUUGCCCUCAG |
| SEQ ID NO 124 | CAAUAGUGGUCAGUCCAGGAGCUAG | SEQ ID NO 147 | UAGGUCAGGCUGCUUUGCCCUCAGC |
| SEQ ID NO 125 | AAUAGUGGUCAGUCCAGGAGCUAGG | SEQ ID NO 148 | AGGUCAGGCUGCUUUGCCCUCAGCU |
| SEQ ID NO 126 | AUAGUGGUCAGUCCAGGAGCUAGGU | SEQ ID NO 149 | GGUCAGGCUGCUUUGCCCUCAGCUC |
| SEQ ID NO 127 **PS248** | AUAGUGGUCAGUCCAGGAGCU | SEQ ID NO 150 | GUCAGGCUGCUUUGCCCUCAGCUCU |
| SEQ ID NO 128 | UAGUGGUCAGUCCAGGAGCUAGGUC | SEQ ID NO 151 | UCAGGCUGCUUUGCCCUCAGCUCUU |
| SEQ ID NO 129 | AGUGGUCAGUCCAGGAGCUAGGUCA | SEQ ID NO 152 | CAGGCUGCUUUGCCCUCAGCUCUUG |
| SEQ ID NO 130 | GUGGUCAGUCCAGGAGCUAGGUCAG | SEQ ID NO 153 | AGGCUGCUUUGCCCUCAGCUCUUGA |
| SEQ ID NO 131 | UGGUCAGUCCAGGAGCUAGGUCAGG | SEQ ID NO 154 | GGCUGCUUUGCCCUCAGCUCUUGAA |
| SEQ ID NO 132 | GGUCAGUCCAGGAGCUAGGUCAGGC | SEQ ID NO 155 | GCUGCUUUGCCCUCAGCUCUUGAAG |
| SEQ ID NO 133 | GUCAGUCCAGGAGCUAGGUCAGGCU | SEQ ID NO 156 | CUGCUUUGCCCUCAGCUCUUGAAGU |
| SEQ ID NO 134 | UCAGUCCAGGAGCUAGGUCAGGCUG | SEQ ID NO 157 | UGCUUUGCCCUCAGCUCUUGAAGUA |
| SEQ ID NO 135 | CAGUCCAGGAGCUAGGUCAGGCUGC | SEQ ID NO 158 | GCUUUGCCCUCAGCUCUUGAAGUAA |
| SEQ ID NO 136 | AGUCCAGGAGCUAGGUCAGGCUGCU | SEQ ID NO 159 | CUUUGCCCUCAGCUCUUGAAGUAAA |
| SEQ ID NO 137 | GUCCAGGAGCUAGGUCAGGCUGCUU | SEQ ID NO 160 | UUUGCCCUCAGCUCUUGAAGUAAAC |
| SEQ ID NO 138 | UCCAGGAGCUAGGUCAGGCUGCUUU | SEQ ID NO 161 | UUGCCCUCAGCUCUUGAAGUAAACG |
| SEQ ID NO 139 | CCAGGAGCUAGGUCAGGCUGCUUUG | SEQ ID NO 162 | UGCCCUCAGCUCUUGAAGUAAACGG |
| SEQ ID NO 140 | CAGGAGCUAGGUCAGGCUGCUUUGC | SEQ ID NO 163 | GCCCUCAGCUCUUGAAGUAAACGGU |
| SEQ ID NO 141 | AGGAGCUAGGUCAGGCUGCUUUGCC | SEQ ID NO 164 | CCCUCAGCUCUUGAAGUAAACGGUU |
| SEQ ID NO 142 | GGAGCUAGGUCAGGCUGCUUUGCCC | SEQ ID NO 165 **PS246** | CCUCAGCUCUUGAAGUAAAC |
| SEQ ID NO 143 | GAGCUAGGUCAGGCUGCUUUGCCCU | SEQ ID NO 166 **PS247** | CCUCAGCUCUUGAAGUAAACG |
| SEQ ID NO 144 | AGCUAGGUCAGGCUGCUUUGCCCUC | SEQ ID NO 167 **PS245** | CUCAGCUCUUGAAGUAAACG |
| SEQ ID NO 145 | GCUAGGUCAGGCUGCUUUGCCCUCA | SEQ ID NO 529 | CCUCAGCUCUUGAAGUAAACGGUUU |
| SEQ ID NO 530 | CUCAGCUCUUGAAGUAAACGGUUUA | SEQ ID NO 531 | UCAGCUCUUGAAGUAAACGGUUUAC |
| SEQ ID NO 532 | CAGCUCUUGAAGUAAACGGUUUACC | SEQ ID NO 533 | AGCUCUUGAAGUAAACGGUUUACCG |
| SEQ ID NO 534 | GCUCUUGAAGUAAACGGUUUACCGC | SEQ ID NO 535 | CUCUUGAAGUAAACGGUUUACCGCC |

**Table 4: oligonucleotides for skipping DMD Gene Exon 51**

| | | | |
|---|---|---|---|
| SEQ ID NO 168 | GUACCUCCAACAUCAAGGAAGAUGG | SEQ ID NO 205 | GAGAUGGCAGUUUCCUUAGUAACCA |
| SEQ ID NO 169 | UACCUCCAACAUCAAGGAAGAUGGC | SEQ ID NO 206 | AGAUGGCAGUUUCCUUAGUAACCAC |
| SEQ ID NO 170 | ACCUCCAACAUCAAGGAAGAUGGCA | SEQ ID NO 207 | GAUGGCAGUUUCCUUAGUAACCACA |
| SEQ ID NO 171 | CCUCCAACAUCAAGGAAGAUGGCAU | SEQ ID NO 208 | AUGGCAGUUUCCUUAGUAACCACAG |
| SEQ ID NO 172 | CUCCAACAUCAAGGAAGAUGGCAUU | SEQ ID NO 209 | UGGCAGUUUCCUUAGUAACCACAGG |
| SEQ ID NO 173 | UCCAACAUCAAGGAAGAUGGCAUUU | SEQ ID NO 210 | GGCAGUUUCCUUAGUAACCACAGGU |
| SEQ ID NO 174 | CCAACAUCAAGGAAGAUGGCAUUUC | SEQ ID NO 211 | GCAGUUUCCUUAGUAACCACAGGUU |
| SEQ ID NO 175 | CAACAUCAAGGAAGAUGGCAUUUCU | SEQ ID NO 212 | CAGUUUCCUUAGUAACCACAGGUUG |
| SEQ ID NO 176 | AACAUCAAGGAAGAUGGCAUUUCUA | SEQ ID NO 213 | AGUUUCCUUAGUAACCACAGGUUGU |
| SEQ ID NO 177 | ACAUCAAGGAAGAUGGCAUUUCUAG | SEQ ID NO 214 | GUUUCCUUAGUAACCACAGGUUGUG |
| SEQ ID NO 178 | CAUCAAGGAAGAUGGCAUUUCUAGU | SEQ ID NO 215 | UUUCCUUAGUAACCACAGGUUGUGU |
| SEQ ID NO 179 | AUCAAGGAAGAUGGCAUUUCUAGUU | SEQ ID NO 216 | UUCCUUAGUAACCACAGGUUGUGUC |
| SEQ ID NO 180 | UCAAGGAAGAUGGCAUUUCUAGUUU | SEQ ID NO 217 | UCCUUAGUAACCACAGGUUGUGUCA |
| SEQ ID NO 181 | CAAGGAAGAUGGCAUUUCUAGUUUG | SEQ ID NO 218 | CCUUAGUAACCACAGGUUGUGUCAC |
| SEQ ID NO 182 | AAGGAAGAUGGCAUUUCUAGUUUGG | SEQ ID NO 219 | CUUAGUAACCACAGGUUGUGUCACC |
| SEQ ID NO 183 | AGGAAGAUGGCAUUUCUAGUUUGGA | SEQ ID NO 220 | UUAGUAACCACAGGUUGUGUCACCA |
| SEQ ID NO 184 | GGAAGAUGGCAUUUCUAGUUUGGAG | SEQ ID NO 221 | UAGUAACCACAGGUUGUGUCACCAG |
| SEQ ID NO 185 | GAAGAUGGCAUUUCUAGUUUGGAGA | SEQ ID NO 222 | AGUAACCACAGGUUGUGUCACCAGA |
| SEQ ID NO 186 | AAGAUGGCAUUUCUAGUUUGGAGAU | SEQ ID NO 223 | GUAACCACAGGUUGUGUCACCAGAG |
| SEQ ID NO 187 | AGAUGGCAUUUCUAGUUUGGAGAUG | SEQ ID NO 224 | UAACCACAGGUUGUGUCACCAGAGU |
| SEQ ID NO 188 | GAUGGCAUUUCUAGUUUGGAGAUGG | SEQ ID NO 225 | AACCACAGGUUGUGUCACCAGAGUA |
| SEQ ID NO 189 | AUGGCAUUUCUAGUUUGGAGAUGGC | SEQ ID NO 226 | ACCACAGGUUGUGUCACCAGAGUAA |
| SEQ ID NO 190 | UGGCAUUUCUAGUUUGGAGAUGGCA | SEQ ID NO 227 | CCACAGGUUGUGUCACCAGAGUAAC |
| SEQ ID NO 191 | GGCAUUUCUAGUUUGGAGAUGGCAG | SEQ ID NO 228 | CACAGGUUGUGUCACCAGAGUAACA |
| SEQ ID NO 192 | GCAUUUCUAGUUUGGAGAUGGCAGU | SEQ ID NO 229 | ACAGGUUGUGUCACCAGAGUAACAG |
| SEQ ID NO 193 | CAUUUCUAGUUUGGAGAUGGCAGUU | SEQ ID NO 230 | CAGGUUGUGUCACCAGAGUAACAGU |
| SEQ ID NO 194 | AUUUCUAGUUUGGAGAUGGCAGUUU | SEQ ID NO 231 | AGGUUGUGUCACCAGAGUAACAGUC |
| SEQ ID NO 195 | UUUCUAGUUUGGAGAUGGCAGUUUC | SEQ ID NO 232 | GGUUGUGUCACCAGAGUAACAGUCU |
| SEQ ID NO 196 | UUCUAGUUUGGAGAUGGCAGUUUCC | SEQ ID NO 233 | GUUGUGUCACCAGAGUAACAGUCUG |
| SEQ ID NO 197 | UCUAGUUUGGAGAUGGCAGUUUCCU | SEQ ID NO 234 | UUGUGUCACCAGAGUAACAGUCUGA |
| SEQ ID NO 198 | CUAGUUUGGAGAUGGCAGUUUCCUU | SEQ ID NO 235 | UGUGUCACCAGAGUAACAGUCUGAG |
| SEQ ID NO 199 | UAGUUUGGAGAUGGCAGUUUCCUUA | SEQ ID NO 236 | GUGUCACCAGAGUAACAGUCUGAGU |
| SEQ ID NO 200 | AGUUUGGAGAUGGCAGUUUCCUUAG | SEQ ID NO 237 | UGUCACCAGAGUAACAGUCUGAGUA |
| SEQ ID NO 201 | GUUUGGAGAUGGCAGUUUCCUUAGU | SEQ ID NO 238 | GUCACCAGAGUAACAGUCUGAGUAG |
| SEQ ID NO 202 | UUUGGAGAUGGCAGUUUCCUUAGUA | SEQ ID NO 239 | UCACCAGAGUAACAGUCUGAGUAGG |
| SEQ ID NO 203 | UUGGAGAUGGCAGUUUCCUUAGUAA | SEQ ID NO 240 | CACCAGAGUAACAGUCUGAGUAGGA |
| SEQ ID NO 204 | UGGAGAUGGCAGUUUCCUUAGUAAC | SEQ ID NO 241 | ACCAGAGUAACAGUCUGAGUAGGAG |

**Table 5: oligonucleotides for skipping DMD Gene Exon 52**

| | | | |
|---|---|---|---|
| SEQ ID NO 242 | AGCCUCUUGAUUGCUGGUCUUGUUU | SEQ ID NO 277 | UUGGGCAGCGGUAAUGAGUUCUUCC |
| SEQ ID NO 243 | GCCUCUUGAUUGCUGGUCUUGUUUU | SEQ ID NO 278 | UGGGCAGCGGUAAUGAGUUCUUCCA |
| SEQ ID NO 244 | CCUCUUGAUUGCUGGUCUUGUUUUU | SEQ ID NO 279 | GGGCAGCGGUAAUGAGUUCUUCCAA |
| SEQ ID NO 245 | CCUCUUGAUUGCUGGUCUUG | SEQ ID NO 280 | GGCAGCGGUAAUGAGUUCUUCCAAC |
| SEQ ID NO 246 **PS232** | CUCUUGAUUGCUGGUCUUGUUUUUC | SEQ ID NO 281 | GCAGCGGUAAUGAGUUCUUCCAACU |
| SEQ ID NO 247 | UCUUGAUUGCUGGUCUUGUUUUUCA | SEQ ID NO 282 | CAGCGGUAAUGAGUUCUUCCAACUG |
| SEQ ID NO 248 | CUUGAUUGCUGGUCUUGUUUUUCAA | SEQ ID NO 283 | AGCGGUAAUGAGUUCUUCCAACUGG |
| SEQ ID NO 249 | UUGAUUGCUGGUCUUGUUUUUCAAA | SEQ ID NO 284 | GCGGUAAUGAGUUCUUCCAACUGGG |
| SEQ ID NO 250 | UGAUUGCUGGUCUUGUUUUUCAAAU | SEQ ID NO 285 | CGGUAAUGAGUUCUUCCAACUGGGG |
| SEQ ID NO 251 | GAUUGCUGGUCUUGUUUUUCAAAUU | SEQ ID NO 286 | GGUAAUGAGUUCUUCCAACUGGGGA |
| SEQ ID NO 252 | GAUUGCUGGUCUUGUUUUUC | SEQ ID NO 287 | GGUAAUGAGUUCUUCCAACUGG |
| SEQ ID NO 253 | AUUGCUGGUCUUGUUUUUCAAAUUU | SEQ ID NO 288 | GUAAUGAGUUCUUCCAACUGGGGAC |
| SEQ ID NO 254 | UUGCUGGUCUUGUUUUUCAAAUUUU | SEQ ID NO 289 | UAAUGAGUUCUUCCAACUGGGGACG |
| SEQ ID NO 255 | UGCUGGUCUUGUUUUUCAAAUUUUG | SEQ ID NO 290 | AAUGAGUUCUUCCAACUGGGGACGC |
| SEQ ID NO 256 | GCUGGUCUUGUUUUUCAAAUUUUGG | SEQ ID NO 291 | AUGAGUUCUUCCAACUGGGGACGCC |
| SEQ ID NO 257 | CUGGUCUUGUUUUUCAAAUUUUGGG | SEQ ID NO 292 | UGAGUUCUUCCAACUGGGGACGCCU |
| SEQ ID NO 258 | UGGUCUUGUUUUUCAAAUUUUGGGC | SEQ ID NO 293 | GAGUUCUUCCAACUGGGGACGCCUC |
| SEQ ID NO 259 | GGUCUUGUUUUUCAAAUUUUGGGCA | SEQ ID NO 294 | AGUUCUUCCAACUGGGGACGCCUCU |
| SEQ ID NO 260 | GUCUUGUUUUUCAAAUUUUGGGCAG | SEQ ID NO 295 | GUUCUUCCAACUGGGGACGCCUCUG |
| SEQ ID NO 261 | UCUUGUUUUUCAAAUUUUGGGCAGC | SEQ ID NO 296 | UUCUUCCAACUGGGGACGCCUCUGU |
| SEQ ID NO 262 | CUUGUUUUUCAAAUUUUGGGCAGCG | SEQ ID NO 297 | UCUUCCAACUGGGGACGCCUCUGUU |
| SEQ ID NO 263 | UUGUUUUUCAAAUUUUGGGCAGCGG | SEQ ID NO 298 | CUUCCAACUGGGGACGCCUCUGUUC |
| SEQ ID NO 264 | UGUUUUUCAAAUUUUGGGCAGCGGU | SEQ ID NO 299 **PS236** | UUCCAACUGGGGACGCCUCUGUUCC |
| SEQ ID NO 265 | GUUUUUCAAAUUUUGGGCAGCGGUA | SEQ ID NO 300 | UCCAACUGGGGACGCCUCUGUUCCA |
| SEQ ID NO 266 | UUUUUCAAAUUUUGGGCAGCGGUAA | SEQ ID NO 301 | CCAACUGGGGACGCCUCUGUUCCAA |
| SEQ ID NO 267 | UUUUCAAAUUUUGGGCAGCGGUAAU | SEQ ID NO 302 | CAACUGGGGACGCCUCUGUUCCAAA |
| SEQ ID NO 268 | UUUCAAAUUUUGGGCAGCGGUAAUG | SEQ ID NO 303 | AACUGGGGACGCCUCUGUUCCAAAU |
| SEQ ID NO 269 | UUCAAAUUUUGGGCAGCGGUAAUGA | SEQ ID NO 304 | ACUGGGGACGCCUCUGUUCCAAAUC |
| SEQ ID NO 270 | UCAAAUUUUGGGCAGCGGUAAUGAG | SEQ ID NO 305 | CUGGGGACGCCUCUGUUCCAAAUCC |
| SEQ ID NO 271 | CAAAUUUUGGGCAGCGGUAAUGAGU | SEQ ID NO 306 | UGGGGACGCCUCUGUUCCAAAUCCU |
| SEQ ID NO 272 | AAAUUUUGGGCAGCGGUAAUGAGUU | SEQ ID NO 307 | GGGGACGCCUCUGUUCCAAAUCCUG |
| SEQ ID NO 273 | AAUUUUGGGCAGCGGUAAUGAGUUC | SEQ ID NO 308 | GGGACGCCUCUGUUCCAAAUCCUGC |
| SEQ ID NO 274 | AUUUUGGGCAGCGGUAAUGAGUUCU | SEQ ID NO 309 | GGACGCCUCUGUUCCAAAUCCUGCA |
| SEQ ID NO 275 | UUUUGGGCAGCGGUAAUGAGUUCUU | SEQ ID NO 310 | GACGCCUCUGUUCCAAAUCCUGCAU |
| SEQ ID NO 276 | UUUGGGCAGCGGUAAUGAGUUCUUC | | |

**Table 6: oligonucleotides for skipping DMD Gene Exon 53**

| | | | |
|---|---|---|---|
| SEQ ID NO 311 | CUCUGGCCUGUCCUAAGACCUGCUC | SEQ ID NO 335 | CAGCUUCUUCCUUAGCUUCCAGCCA |
| SEQ ID NO 312 | UCUGGCCUGUCCUAAGACCUGCUCA | SEQ ID NO 336 | AGCUUCUUCCUUAGCUUCCAGCCAU |
| SEQ ID NO 313 | CUGGCCUGUCCUAAGACCUGCUCAG | SEQ ID NO 337 | GCUUCUUCCUUAGCUUCCAGCCAUU |
| SEQ ID NO 314 | UGGCCUGUCCUAAGACCUGCUCAGC | SEQ ID NO 338 | CUUCUUCCUUAGCUUCCAGCCAUUG |
| SEQ ID NO 315 | GGCCUGUCCUAAGACCUGCUCAGCU | SEQ ID NO 339 | UUCUUCCUUAGCUUCCAGCCAUUGU |
| SEQ ID NO 316 | GCCUGUCCUAAGACCUGCUCAGCUU | SEQ ID NO 340 | UCUUCCUUAGCUUCCAGCCAUUGUG |
| SEQ ID NO 317 | CCUGUCCUAAGACCUGCUCAGCUUC | SEQ ID NO 341 | CUUCCUUAGCUUCCAGCCAUUGUGU |
| SEQ ID NO 318 | CUGUCCUAAGACCUGCUCAGCUUCU | SEQ ID NO 342 | UUCCUUAGCUUCCAGCCAUUGUGUU |
| SEQ ID NO 319 | UGUCCUAAGACCUGCUCAGCUUCUU | SEQ ID NO 343 | UCCUUAGCUUCCAGCCAUUGUGUUG |
| SEQ ID NO 320 | GUCCUAAGACCUGCUCAGCUUCUUC | SEQ ID NO 344 | CCUUAGCUUCCAGCCAUUGUGUUGA |
| SEQ ID NO 321 | UCCUAAGACCUGCUCAGCUUCUUCC | SEQ ID NO 345 | CUUAGCUUCCAGCCAUUGUGUUGAA |
| SEQ ID NO 322 | CCUAAGACCUGCUCAGCUUCUUCCU | SEQ ID NO 346 | UUAGCUUCCAGCCAUUGUGUUGAAU |
| SEQ ID NO 323 | CUAAGACCUGCUCAGCUUCUUCCUU | SEQ ID NO 347 | UAGCUUCCAGCCAUUGUGUUGAAUC |
| SEQ ID NO 324 | UAAGACCUGCUCAGCUUCUUCCUUA | SEQ ID NO 348 | AGCUUCCAGCCAUUGUGUUGAAUCC |
| SEQ ID NO 325 | AAGACCUGCUCAGCUUCUUCCUUAG | SEQ ID NO 349 | GCUUCCAGCCAUUGUGUUGAAUCCU |
| SEQ ID NO 326 | AGACCUGCUCAGCUUCUUCCUUAGC | SEQ ID NO 350 | CUUCCAGCCAUUGUGUUGAAUCCUU |
| SEQ ID NO 327 | GACCUGCUCAGCUUCUUCCUUAGCU | SEQ ID NO 351 | UUCCAGCCAUUGUGUUGAAUCCUUU |
| SEQ ID NO 328 | ACCUGCUCAGCUUCUUCCUUAGCUU | SEQ ID NO 352 | UCCAGCCAUUGUGUUGAAUCCUUUA |
| SEQ ID NO 329 | CCUGCUCAGCUUCUUCCUUAGCUUC | SEQ ID NO 353 | CCAGCCAUUGUGUUGAAUCCUUUAA |
| SEQ ID NO 330 | CUGCUCAGCUUCUUCCUUAGCUUCC | SEQ ID NO 354 | CAGCCAUUGUGUUGAAUCCUUUAAC |
| SEQ ID NO 331 | UGCUCAGCUUCUUCCUUAGCUUCCA | SEQ ID NO 355 | AGCCAUUGUGUUGAAUCCUUUAACA |
| SEQ ID NO 332 | GCUCAGCUUCUUCCUUAGCUUCCAG | SEQ ID NO 356 | GCCAUUGUGUUGAAUCCUUUAACAU |
| SEQ ID NO 333 | CUCAGCUUCUUCCUUAGCUUCCAGC | SEQ ID NO 357 | CCAUUGUGUUGAAUCCUUUAACAUU |
| SEQ ID NO 334 | UCAGCUUCUUCCUUAGCUUCCAGCC | SEQ ID NO 358 | CAUUGUGUUGAAUCCUUUAACAUUU |

**Table 7 : oligonucleotides for skipping other exons of the DMD gene as identified**

| **DMD Gene Exon 6** | | | |
|---|---|---|---|
| SEQ ID NO 359 | CAUUUUUGACCUACAUGUGG | SEQ ID NO 364 | AUUUUUGACCUACAUGGGAAA G |
| SEQ ID NO 360 | UUUGACCUACAUGUGGAAAG | SEQ ID NO 365 | UACGAGUUGAUUGUCGGACCCAG |
| SEQ ID NO 361 | UACAUUUUUGACCUACAUGUGGAA AG | SEQ ID NO 366 | GUGGUCUCCUUACCUAUGACUGUGG |
| SEQ ID NO 362 | GGUCUCCUUACCUAUGA | SEQ ID NO 367 | UGUCUCAGUAAUCUUCUUACCUAU |
| SEQ ID NO 363 | UCUUACCUAUGACUAUGGAUGAGA | | |
| | | | |

| **DMD Gene Exon 7** | | | |
|---|---|---|---|
| SEQ ID NO368 | UGCAUGUUCCAGUCGUUGUGUGG | SEQ ID NO 370 | AUUUACCAACCUUCAGGAUCGAGU A |
| SEQ ID NO 369 | CACUAUUCCAGUCAAAUAGGUCUGG | SEQ ID NO 371 | GGCCUAAAACACAUACACAUA |
| | | | |

| **DMD Gene Exon 11** | | | |
|---|---|---|---|
| SEQ ID NO 372 | CCCUGAGGCAUUCCCAUCUUGAAU | SEQ ID NO 374 | CUUGAAUUUAGGAGAUUCAUCU |
| | | | G |
| SEQ ID NO 373 | AGGACUUACUUGCUUUGUUU | SEQ ID NO 375 | CAUCUUCUGAUAAUUUUCCUGUU |
| | | | |

| **DMD Gene Exon 17** | | | |
|---|---|---|---|
| SEQ ID NO 376 | CCAUUACAGUUGUCUGUGUU | SEQ ID NO 378 | UAAUCUGCCUCUUCUUUUGG |
| SEQ ID NO 377 | UGACAGCCUGUGAAAUCUGUGAG | | |
| | | | |

| **DMD Gene Exon 19** | | | |
|---|---|---|---|
| SEQ ID NO 379 | CAGCAGUAGUUGUCAUCUGC | SEQ ID NO 381 | GCCUGAGCUGAUCUGCUGGCAUC UUGCAGUU |
| SEQ ID NO 380 | GCCUGAGCUGAUCUGCUGGCAUCUUGC | SEQ ID NO 382 | UCUGCUGGCAUCUUGC |
| | | | |

| **DMD Gene Exon 21** | | | |
|---|---|---|---|
| SEQ ID NO 383 | GCCGGUUGACUUCAUCCUGUGC | SEQ ID NO 386 | CUGCAUCCAGGAACAUGGGUCC |
| SEQ ID NO 384 | GUCUGCAUCCAGGAACAUGGGUC | SEQ ID NO 387 | GUUGAAGAUCUGAUAGCCGGUU GA |
| SEQ ID NO 385 | UACUUACUGUCUGUAGCUCUUUCU | | |
| | | | |

| **DMD Gene Exon 44** | | | |
|---|---|---|---|
| SEQ ID NO 388 | UCAGCUUCUGUUAGCCACUG | SEQ ID NO 413 | AGCUUCUGUUAGCCACUGAUUAA A |
| SEQ ID NO 389 | UUCAGCUUCUGUUAGCCACU | SEQ ID NO 414 | CAGCUUCUGUUAGCCACUGAUUA AA |
| SEQ ID NO 390 | UUCAGCUUCUGUUAGCCACUG | SEQ ID NO 415 | AGCUUCUGUUAGCCACUGAUUAA A |
| SEQ ID NO 391 | UCAGCUUCUGUUAGCCACUGA | SEQ ID NO 416 | AGCUUCUGUUAGCCACUGAU |
| SEQ ID NO 392 | UUCAGCUUCUGUUAGCCACUGA | SEQ ID NO 417 | GCUUCUGUUAGCCACUGAUU |
| SEQ ID NO 393 | UCAGCUUCUGUUAGCCACUGA | SEQ ID NO 418 | AGCUUCUGUUAGCCACUGAUU |
| SEQ ID NO 394 | UUCAGCUUCUGUUAGCCACUGA | SEQ ID NO 419 | GCUUCUGUUAGCCACUGAUUA |
| SEQ ID NO 395 | UCAGCUUCUGUUAGCCACUGAU | SEQ ID NO 420 | AGCUUCUGUUAGCCACUGAUUA |
| SEQ ID NO 396 | UUCAGCUUCUGUUAGCCACUGAU | SEQ ID NO 421 | GCUUCUGUUAGCCACUGAUUAA |
| SEQ ID NO 397 | UCAGCUUCUGUUAGCCACUGAUU | SEQ ID NO 422 | AGCUUCUGUUAGCCACUGAUUAA |
| SEQ ID NO 398 | UUCAGCUUCUGUUAGCCACUGAUU | SEQ ID NO 423 | GCUUCUGUUAGCCACUGAUUAAA |
| SEQ IDNO 399 | UCAGCUUCUGUUAGCCACUGAUUA | SEQ ID NO 424 | AGCUUCUGUUAGCCACUGAUUAA |
| | | | A |
| SEQ ID NO 400 | UUCAGCUUCUGUUAGCCACUGAUA | SEQ ID NO 425 | GCUUCUGUUAGCCACUGAUUAAA |
| SEQ ID NO 401 | UCAGCUUCUGUUAGCCACUGAUUAA | SEQ ID NO 426 | CCAUUUGUAUUUAGCAUGUUCCC |
| SEQ ID NO 402 | UUCAGCUUCUGUUAGCCACUGAUUAA | SEQ ID NO 427 | AGAUACCAUUUGUAUUUAGC |
| SEQ ID NO 403 | UCAGCUUCUGUUAGCCACUGAUUAAA | SEQ ID NO 428 | GCCAUUUCUCAACAGAUCU |
| SEQ ID NO 404 | UUCAGCUUCUGUUAGCCACUGAUUAAA | SEQ ID NO 429 | GCCAUUUCUCAACAGAUCUGUCA |
| SEQ ID NO 405 | CAGCUUCUGUUAGCCACUG | SEQ ID NO 430 | AUUCUCAGGAAUUUGUGUCUUUC |
| SEQ ID NO 406 | CAGCUUCUGUUAGCCACUGAU | SEQ ID NO 431 | UCUCAGGAAUUUGUGUCUUUC |
| SEQ ID NO 407 | AGCUUCUGUUAGCCACUGAUU | SEQ ID NO 432 | GUUCAGCUUCUGUUAGCC |
| SEQ ID NO 408 | CAGCUUCUGUUAGCCACUGAUU | SEQ ID NO 433 | CUGAUUAAAUAUCUUUAUAU C |
| SEQ ID NO 409 | AGCUUCUGUUAGCCACUGAUUA | SEQ ID NO 434 | GCCGCCAUUUCUCAACAG |
| SEQ ID NO 410 | CAGCUUCUGUUAGCCACUGAUUA | SEQ ID NO 435 | GUAUUUAGCAUGUUCCCA |
| SEQ ID NO 411 | AGCUUCUGUUAGCCACUGAUUAA | SEQ ID NO 436 | CAGGAAUUUGUGUCUUUC |
| SEQ ID NO 412 | CAGCUUCUGUUAGCCACUGAUUAA | | |
| | | | |

| **DMD Gene Exon 45** | | | |
|---|---|---|---|
| SEQ ID NO 437 | UUUGCCGCUGCCCAAUGCCAUCCUG | SEQ ID NO 470 | GUUGCAUUCAAUGUUCUGACAAC AG |
| SEQ ID NO 438 | AUUCAAUGUUCUGACAACAGUUUGC | SEQ ID NO 471 | UUGCAUUCAAUGUUCUGACAACA GU |
| SEQ ID NO 439 | CCAGUUGCAUUCAAUGUUCUGACAA | SEQ ID NO 472 | UGCAUUCAAUGUUCUGACAACAG UU |
| SEQ ID NO 440 | CAGUUGCAUUCAAUGUUCUGAC | SEQ ID NO 473 | GCAUUCAAUGUUCUGACAACAGU UU |
| SEQ ID NO 441 | AGUUGCAUUCAAUGUUCUGA | SEQ ID NO 474 | CAUUCAAUGUUCUGACAACAGUU UG |
| SEQ ID NO 442 | GAUUGCUGAAUUAUUUCUUCC | SEQ ID NO 475 | AUUCAAUGUUCUGACAACAGUUU GC |
| SEQ ID NO 443 | GAUUGCUGAAUUAUUUCUUCCCCAG | SEQ ID NO 476 | UCAAUGUUCUGACAACAGUUUGC CG |
| SEQ ID NO 444 | AUUGCUGAAUUAUUUCUUCCCCAGU | SEQ ID NO 477 | CAAUGUUCUGACAACAGUUUGCC GC |
| SEQ ID NO 445 | UUGCUGAAUUAUUUCUUCCCCAGUU | SEQ ID NO 478 | AAUGUUCUGACAACAGUUUGCCG CU |
| SEQ ID NO 446 | UGCUGAAUUAUUUCUUCCCCAGUUG | SEQ ID NO 479 | AUGUUCUGACAACAGUUUGCCGC UG |
| SEQ ID NO 447 | GCUGAAUUAUUUCUUCCCCAGUUGC | SEQ ID NO 480 | UGUUCUGACAACAGUUUGCCGCU GC |
| SEQ ID NO 448 | CUGAAUUAUUUCUUCCCCAGUUGCA | SEQ ID NO 481 | GUUCUGACAACAGUUUGCCGCUG CC |
| SEQ ID NO 449 | UGAAUUAUUUCUUCCCCAGUUGCAU | SEQ ID NO 482 | UUCUGACAACAGUUUGCCGCUGC CC |
| SEQ ID NO 450 | GAAUUAUUUCUUCCCCAGUUGCAUU | SEQ ID NO 483 | UCUGACAACAGUUUGCCGCUGCC CA |
| SEQ ID NO 451 | AAUUAUUUCUUCCCCAGUUGCAUUC | SEQ ID NO 484 | CUGACAACAGUUUGCCGCUGCCC AA |
| SEQ ID NO 452 | AUUAUUUCUUCCCCAGUUGCAUUCA | SEQ ID NO 485 | UGACAACAGUUUGCCGCUGCCCA AU |
| SEQ ID NO 453 | UUAUUUCUUCCCCAGUUGCAUUCAA | SEQ ID NO 486 | GACAACAGUUUGCCGCUGCCCAA UG |
| SEQ ID NO 454 | UAUUUCUUCCCCAGUUGCAUUCAAU | SEQ ID NO 487 | ACAACAGUUUGCCGCUGCCCAAU GC |
| SEQ ID NO 455 | AUUUCUUCCCCAGUUGCAUUCAAUG | SEQ ID NO 488 | CAACAGUUUGCCGCUGCCCAAUG CC |
| SEQ ID NO 456 | UUUCUUCCCCAGUUGCAUUCAAUGU | SEQ ID NO 489 | AACAGUUUGCCGCUGCCCAAUGC CA |
| SEQ ID NO 457 | UUCUUCCCCAGUUGCAUUCAAUGUU | SEQ ID NO 490 | ACAGUUUGCCGCUGCCCAAUGCC AU |
| SEQ ID NO 458 | UCUUCCCCAGUUGCAUUCAAUGUUC | SEQ ID NO 491 | CAGUUUGCCGCUGCCCAAUGCCA UC |
| SEQ ID NO 459 | CUUCCCCAGUUGCAUUCAAUGUUCU | SEQ ID NO 492 | AGUUUGCCGCUGCCCAAUGCCAU CC |
| SEQ ID NO 460 | UUCCCCAGUUGCAUUCAAUGUUCUG | SEQ ID NO 493 | GUUUGCCGCUGCCCAAUGCCAUC CU |
| SEQ ID NO 461 | UCCCCAGUUGCAUUCAAUGUUCUGA | SEQ ID NO 494 | UUUGCCGCUGCCCAAUGCCAUCC UG |
| SEQ ID NO 462 | CCCCAGUUGCAUUCAAUGUUCUGAC | SEQ ID NO 495 | UUGCCGCUGCCCAAUGCCAUCCU GG |
| SEQ ID NO 463 | CCCAGUUGCAUUCAAUGUUCUGACA | SEQ ID NO 496 | UGCCGCUGCCCAAUGCCAUCCUG GA |
| SEQ ID NO 464 | CCAGUUGCAUUCAAUGUUCUGACAA | SEQ ID NO 497 | GCCGCUGCCCAAUGCCAUCCUGG AG |
| SEQ ID NO 465 | CAGUUGCAUUCAAUGUUCUGACAAC | SEQ ID NO 498 | CCGCUGCCCAAUGCCAUCCUGGA GU |
| SEQ ID NO 466 | AGUUGCAUUCAAUGUUCUGACAACA | SEQ ID NO 499 | CGCUGCCCAAUGCCAUCCUGGAG UU |
| SEQ ID NO 467 | UCC UGU AGA AUA CUG GCA UC | SEQ ID NO 500 | UGUUUUUGAGGAUUGCUGAA |
| SEQ ID NO 468 | UGCAGACCUCCUGCCACCGCAGAUUCA | SEQ ID NO 501 | UGUUCUGACAACAGUUUGCCGCU GCCCAAUGCCAUCCUGG |
| SEQ ID NO 469 | UUGCAGACCUCCUGCCACCGCAGAUUCAGGC UUC | | |
| | | | |

| **DMD Gene Exon 55** | | | |
|---|---|---|---|
| SEQ ID NO 502 | CUGUUGCAGUAAUCUAUGAG | SEQ ID | UGCCAUUGUUUCAUCAGCUCUUU |
| | | NO 505 | |
| SEQ ID NO 503 | UGCAGUAAUCUAUGAGUUUC | SEQ ID NO 506 | UCCUGUAGGACAUUGGCAGU |
| SEQ ID NO 504 | GAGUCUUCUAGGAGCCUU | SEQ ID NO 507 | CUUGGAGUCUUCUAGGAGCC |
| | | | |

| **DMD Gene Exon 57** | | | |
|---|---|---|---|
| SEQ ID NO 508 | UAGGUGCCUGCCGGCUU | SEQ ID NO 510 | CUGAACUGCUGGAAAGUCGCC |
| SEQ ID NO 509 | UUCAGCUGUAGCCACACC | SEQ ID NO 511 | CUGGCUUCCAAAUGGGACCUGAA AAAGAAC |
| | | | |

| **DMD Gene Exon 59** | | | |
|---|---|---|---|
| SEQ ID NO 512 | CAAUUUUUCCCACUCAGUAUU | SEQ ID NO 514 | UCCUCAGGAGGCAGCUCUAAAU |
| SEQ ID NO 513 | UUGAAGUUCCUGGAGUCUU | | |
| | | | |

| **DMD Gene Exon 62** | | | |
|---|---|---|---|
| SEQ ID NO 515 | UGGCUCUCUCCCAGGG | SEQ ID NO 517 | GGGCACUUUGUUUGGCG |
| SEQ ID NO 516 | GAGAUGGCUCUCUCCCAGGGACCCUGG | | |
| | | | |

| **DMD Gene Exon 63** | | | |
|---|---|---|---|
| SEQ ID NO 518 | GGUCCCAGCAAGUUGUUUG | SEQ ID NO 520 | GUAGAGCUCUGUCAUUUUGGG |
| SEQ ID NO 519 | UGGGAUGGUCCCAGCAAGUUGUUUG | | |
| | | | |

| **DMD Gene Exon 65** | | | |
|---|---|---|---|
| SEQ ID NO 521 | GCUCAAGAGAUCCACUGCAAAAAAC | SEQ ID NO 523 | UCUGCAGGAUAUCCAUGGGCUGG UC |
| SEQ ID NO 522 | GCCAUACGUACGUAUCAUAAACAUUC | | |

| **DMD Gene Exon 66** | | | |
|---|---|---|---|
| SEQ ID NO 524 | GAUCCUCCCUGUUCGUCCCCUAUUAUG | | |
| | | | |

| **DMD Gene Exon 69** | | | |
|---|---|---|---|
| SEQ ID NO 525 | UGCUUUAGACUCCUGUACCUGAUA | | |
| | | | |

| **DMD Gene Exon 75** | | | |
|---|---|---|---|
| SEQ ID NO 526 | GGCGGCCUUUGUGUUGAC | SEQ ID NO 528 | CCUUUAUGUUCGUGCUGCU |
| SEQ ID NO 527 | GGACAGGCCUUUAUGUUCGUGCUGC | | |

### Figure legends

**Figure 1****.** In human control myotubes, a series of AONs (PS237, PS238, and PS240; SEQ ID NO 65, 66, 16 respectively) targeting exon 43 was tested at 500 nM. PS237 (SEQ ID NO 65) reproducibly induced highest levels of exon 43 skipping. (M: DNA size marker; NT: non-treated cells)
**Figure 2****.** In myotubes from a DMD patient with an exon 45 deletion, a series of AONs (PS177, PS179, PS181, and PS182; SEQ ID NO 91, 70, 110, and 117 respectively) targeting exon 46 was tested at two different concentrations (50 and 150 nM). PS182 (SEQ ID NO 117) reproducibly induced highest levels of exon 46 skipping. (M: DNA size marker)
**Figure 3****.** In human control myotubes, a series of AONs (PS245, PS246, PS247, and PS248; SEQ ID NO 167, 165, 166, and 127 respectively) targeting exon 50 was tested at 500 nM. PS248 (SEQ ID NO 127) reproducibly induced highest levels of exon 50 skipping. (M: DNA size marker; NT: non-treated cells).
**Figure 4****.** In human control myotubes, two novel AONs (PS232 and PS236; SEQ ID NO 246 and 299 respectively) targeting exon 52 were tested at two different concentrations (200 and 500 nM) and directly compared to a previously described AON (52-1). PS236 (SEQ ID NO 299) reproducibly induced highest levels of exon 52 skipping. (M: DNA size marker; NT: non-treated cells).

## Claims

1. An antisense oligonucleotide, wherein said antisense oligonucleotide comprises or consists of the antisense nucleotide sequence SEQ ID NO: 287.

2. An oligonucleotide according to claim 1, comprising a 2'-0-alkyl phosphorothioate antisense oligonucleotide.

3. An oligonucleotide according to claim 2, comprising a 2'-0 methyl phosphorothioate ribose.

4. An oligonucleotide according to claim 1 wherein the oligonucleotide is a phosphorodiamidate morpholino oligomer (PMO).

5. A viral-based vector, comprising an expression cassette that drives expression of an oligonucleotide as defined in claim 1.

6. An oligonucleotide according to any one of claims 1 to 4 or the viral-based vector according to claim 5 for use as a medicament, preferably for modulating splicing of the dystrophin pre-mRNA of a DMD or BMD patient or for the treatment of a DMD or BMD patient.

7. A pharmaceutical composition comprising an oligonucleotide as defined in any one of claims 1 to 4 and/or the vector of claim 5, a pharmaceutical acceptable carrier, and optionally combined with an antisense oligonucleotide which is able to induce or promote skipping of at least one of exon 51, 53, 55, 57, 59 or 69 of the dystrophin premRNA of a patient.

8. An in vitro or ex vivo method for inducing and/or promoting skipping of at least exon 52 of the dystrophin pre-mRNA in an isolated cell of a patient, the method comprising providing said cell with an oligonucleotide as defined in any one of claims 1 to 4 or the vector of claim 5.

9. A method according to claim 8, wherein an additional antisense oligonucleotide is used which is able to induce or promote skipping of at least one of exon 51, 53, 55, 57, 59 or 69 of the dystrophin pre-mRNA of a patient.

10. Use of the oligonucleotide as defined in any one of claims 1 to 4, the vector of claim 5, or the pharmaceutical composition of claim 7 for modulating splicing of the dystrophin pre-mRNA in a human myogenic cell or muscle cell in vitro, or for the preparation of a medicament for the treatment of a DMD or BMD patient.

## Patentansprüche

1. Antisense-Oligonukleotid, wobei das Antisense-Oligonukleotid die Antisense-Nukleotidsequenz SEQ ID NR: 287 umfasst oder daraus besteht.

2. Oligonukleotid nach Anspruch 1, umfassend ein 2'-O-Alkylphosphorothioat-Antisense-Oligonukleotid.

3. Oligonukleotid nach Anspruch 2, umfassend eine 2'-Methylphosphorthioat-Ribose.

4. Oligonukleotid nach Anspruch 1, wobei das Oligonukleotid ein Phosphorodiamidat-Morpholino-Oligomer (PMO) ist.

5. Virus-basierter Vektor, umfassend eine Expressionskassette, welche die Expression eines gemäß Anspruch 1 definierten Oligonukleotids steuert.

6. Oligonukleotid nach einem der Ansprüche 1 bis 4 oder Virus-basierter Vektor nach Anspruch 5 zur Verwendung als Medikament, vorzugsweise zur Modulation des Spleißens der Dystrophin-prä-mRNS eines DMD- oder BMD-Patienten oder zur Behandlung eines DMD- oder BMD-Patienten.

7. Pharmazeutische Zusammensetzung, umfassend ein Oligonukleotid wie in einem der Ansprüche 1 bis 4 definiert und/oder den Vektor nach Anspruch 5, einen pharmazeutisch verträglichen Träger, und gegebenenfalls kombiniert mit einem Antisense-Oligonukleotid, das in der Lage ist, mindestens ein Überspringen eines der Exonen 51, 53, 55, 57, 59 oder 69 der Dystrophin-prä-mRNS eines Patienten zu induzieren oder zu fördern.

8. In-vitro- oder Ex-vivo-Verfahren zum Induzieren und/oder Fördern eines Überspringens von zumindest Exon 52 der Dystrophin-prä-mRNS in einer isolierten Zelle eines Patienten, wobei das Verfahren ein Bereitstellen der Zelle mit einem Oligonukleotid, wie in einem der Ansprüche 1 bis 4 definiert, oder dem Vektor gemäß Anspruch 5 umfasst.

9. Verfahren nach Anspruch 8, wobei ein zusätzliches Antisense-Oligonukleotid verwendet wird, das in der Lage ist, das Überspringen von mindestens einem der Exonen 51, 53, 55, 57, 59 oder 69 der Dystrophin-prä-mRNS eines Patienten zu induzieren oder zu fördern.

10. Verwenden des Oligonukleotids nach einem der Ansprüche 1 bis 4, des Vektors nach Anspruch 5 oder der pharmazeutische Zusammensetzung nach Anspruch 7 zur Modulation des Spleißens der Dystrophin-prä-mRNS in vitro in einer menschlichen myogenen Zelle oder einer Muskelzelle, oder zur Herstellung eines Medikaments zur Behandlung eines DMD- oder BMD-Patienten.

## Revendications

1. Oligonucléotide antisens, dans lequel ledit oligonucléotide antisens comprend ou consiste en la séquence de nucléotides antisens SEQ ID NO : 287.

2. Oligonucléotide selon la revendication 1, comprenant un oligonucléotide antisens 2'-O-alkylphosphorothioate.

3. Oligonucléotide selon la revendication 2, comprenant un ribose 2'-O-méthylphosphorothioate.

4. Oligonucléotide selon la revendication 1, dans lequel ledit oligonucléotide est un oligomère morpholino phosphorodiamidate (PMO).

5. Vecteur viral, comprenant une cassette d'expression entraînant l'expression d'un oligonucléotide tel que défini à la revendication 1.

6. Oligonucléotide selon l'une quelconque des revendications 1 à 4 ou vecteur viral selon la revendication 5 destiné à être utilisé en tant que médicament, de préférence pour une modulation de l'épissage de l'ARN pré-messager de la dystrophine d'un patient DMD ou BMD ou pour le traitement d'un patient DMD ou BMD.

7. Composition pharmaceutique comprenant un oligonucléotide tel que défini selon l'une quelconque des revendications 1 à 4 et/ou le vecteur selon la revendication 5, un excipient pharmaceutiquement acceptable, et éventuellement combiné avec un oligonucléotide antisens qui est capable d'induire ou de favoriser le saut d'au moins un exon 51, 53, 55, 57, 59 ou 69 de l'ARN pré-messager de la dystrophine d'un patient.

8. Procédé in vitro ou ex vivo destiné à induire et/ou à favoriser un saut d'au moins l'exon 52 de l'ARN pré-messager de la dystrophine dans une cellule isolée d'un patient, le procédé comprenant une fourniture de ladite cellule avec un oligonucléotide tel que défini selon l'une quelconque des revendications 1 à 4 ou un vecteur selon la revendication 5.

9. Procédé selon la revendication 8, dans lequel on utilise un oligonucléotide antisens supplémentaire qui est capable d'induire ou de favoriser un saut d'au moins un exon 51, 53, 55, 57, 59 ou 69 de l'ARN pré-messager de la dystrophine d'un patient.

10. Utilisation de l'oligonucléotide tel que défini selon l'une quelconque des revendications 1 à 4, du vecteur selon la revendication 5, ou de la composition pharmaceutique selon la revendication 7 pour une modulation de l'épissage de l'ARN pré-messager de la dystrophine dans une cellule myogénique ou une cellule musculaire in vitro, ou pour la préparation d'un médicament pour le traitement d'un patient DMB ou BMD.
